# FASCICULE DE BREVET EUROPEEN

(11) **EP 2 015 549 B1**
(45) Date de publication et mention de la délivrance du brevet: **05.08.2015**
(21) Numéro de dépôt: 08290603.3
(22) Date de dépôt: 25.06.2008
(51) Int. Cl.: H04M 1/725, A61B 5/00

(54) **Procédé et dispositif portable d'alerte autonome en situation demobilité évolutifs**
Verfahren und tragbare Vorrichtung zur autonomen Alarmaktivierung in entwicklungsfähigen Mobilitätssituationen
Upgradeable autonomous warning portable device and method in mobility situation

(30) Priorité: 11.07.2007 FR 0705021
(43) Date de publication de la demande: 14.01.2009
(73) Titulaire: Société Française du Radiotéléphone, 75008 Paris (FR)
(72) Inventeur: Bulian, Fabrice, 94450 Limeil-Brevannes (FR); Teimoorzadeh, Kourosh, 28130 Bouglainval (FR)
(74) Mandataire: Debay, Yves

(56) Documents cités:
- WO-A-2005/027488
- DE-A1- 4 441 907
- US-A1- 2003 236 100
- US-A1- 2004 068 196
- US-A1- 2004 266 480
- US-A1- 2005 066 282
- US-B1- 6 366 871

## Description

Le domaine de la présente invention est celui des télécommunications. Plus particulièrement, il s'agit d'un dispositif portable autonome en situation de mobilité et d'un procédé d'émission d'un ou plusieurs messages d'alerte en réponse à la détection d'un ou plusieurs événements.

Un téléphone portable peut être utilisé comme un outil de sécurité, grâce à sa fonction de mobilité, associée à sa fonction de télécommunication. Les téléphones portables évolués fournissent plusieurs fonctions permettant d'augmenter l'interactivité et permettent ainsi la transmission de courrier sous forme de texte, la transmission de photos ou la transmission de films. En plus du réseau de téléphonie mobile, un téléphone portable est susceptible de communiquer par d'autres réseaux, en utilisant des interfaces de communication supplémentaires, comme par exemple, Bluetooth® ou Wi-Fi.

A ce sujet il est fait référence aux documents US 2004/068 196, US 2003/236 100, US 2005/066282, US 6 366 871, WO 2005 027 488, US 2004/266 480 et DE 44 41 907.

Un problème technique important rencontré dans ce domaine est cependant relatif au fait qu'une procédure pour réaliser un appel d'urgence est souvent une procédure précise, figée et adaptée à une situation déterminée, et souvent inadaptée pour des usages particuliers de l'utilisateur. Le téléphone portable se révèle en effet, souvent inopérant dans des situations d'urgence face à un agresseur ou lorsque l'utilisateur est soumis à un stress important ou encore lorsque l'utilisateur est physiquement blessé. De plus les situations d'urgence rencontrées par l'utilisateur sont susceptibles d'évoluer et nécessitent une adaptabilité fine des procédures d'alerte pour que ces dernières soient authentiquement efficaces et aisées d'utilisation.

La présente invention a pour objet de supprimer un ou plusieurs des inconvénients de l'art antérieur en proposant un procédé évolutif d'émission d'un ou plusieurs messages d'alerte en réponse à la détection d'un ou plusieurs évènements, permettant un paramétrage simple d'une ou plusieurs procédures d'urgence.

Cet objectif est atteint grâce à un procédé d'alerte exécuté par un dispositif portable en situation de mobilité associé à au moins un réseau de télécommunication, le dispositif portable comprenant au moins des moyens de traitement, des moyens de mémorisation et des moyens de réception ou d'émission de données par le réseau, caractérisé en ce qu'il comprend au moins :
- une étape d'initialisation d'au moins un module d'analyse, initialisé en fonction de données représentatives d'un contexte d'utilisation déterminé comprenant au moins des données représentatives d'au moins un état d'au moins un capteur, le module d'analyse comprenant au moins un module d'évaluation évaluant au moins une variable d'alerte,
- une étape d'initialisation d'au moins un module de surveillance initialisé en fonction du contexte d'utilisation, le module de surveillance réglant la variable d'alerte évaluée dans un état représentatif de l'état dudit capteur ou d'un autre capteur, associé au module de surveillance,
- une étape d'initialisation d'au moins un module d'émission d'alerte initialisé en fonction du contexte d'utilisation, commandé en fonction de l'évaluation de la variable d'alerte par le module d'évaluation et déclenchant, lors de son activation, au moins l'envoi d'un message d'alerte par les moyens d'émission de données par le réseau ou par d'autres moyens d'émission,
- une étape de réglage préliminaire à l'activation, d'au moins le module d'évaluation, de surveillance ou d'émission d'alerte, le réglage étant réalisé par l'interface utilisateur,
- une étape d'activation du module d'analyse et de son module d'évaluation,
- une étape de réglage de la variable d'alerte par le module de surveillance associé au contexte,
- une étape d'activation du module d'émission d'alerte lorsque le module d'évaluation détecte un état d'urgence.

Selon une autre particularité, l'étape d'initialisation du module d'analyse, du module de surveillance ou du module d'alerte réalisée en fonction d'au moins une donnée représentative du contexte d'utilisation, comprend la comparaison d'au moins cette donnée avec des données d'initialisation associées à des modules d'une bibliothèque.

Selon une autre particularité, le procédé comprend une étape d'archivage du module d'analyse, du module de surveillance ou respectivement du module d'émission d'alerte, dans la bibliothèque de modules, comprenant le stockage de données représentatives du module d'analyse, du module de surveillance ou respectivement du module d'émission d'alerte, associées à des données représentatives d'un nom entré par l'utilisateur ou de données représentatives du contexte d'utilisation.

Un autre objectif est de proposer un procédé d'alerte permettant de capitaliser de l'expérience pour s'adapter à des situations au cas par cas en temps réel.

Selon cet objectif, l'activation du module d'analyse est réalisée concomitante au stockage, par un module d'apprentissage, de séquences d'évènements informatiques représentatives des états du module d'analyse, de surveillance et d'émission d'alerte, en concordance de phase avec les états des variables d'alerte.

Selon une autre particularité, l'étape d'activation du module d'analyse et de son module d'évaluation est suivie d'au moins une étape de réglage du module d'analyse, du module de surveillance ou du module d'émission d'alerte, en fonction de :
- une commande de paramétrage manuel entrée par une interface utilisateur, ou
- une commande de paramétrage automatique produite par le module d'apprentissage en fonction d'une annulation de l'état d'urgence entrée par l'interface utilisateur.

Selon une autre particularité, le réglage du module d'analyse comprend le réglage de son module d'évaluation par :
- un choix d'un programme d'évaluation dans une bibliothèque de programmes, ou
- un paramétrage du programme d'évaluation choisi, ou
- une association d'un ou plusieurs modules d'émission d'alerte commandés en fonction de l'évaluation, ou
- un réglage d'une ou plusieurs références de variables d'alerte lues en entrée du programme d'évaluation.

Selon une autre particularité, le réglage du module de surveillance comprend:
- un réglage d'une référence d'un capteur surveillé, ou
- un choix d'une fonction de traitement préliminaire réglant la variable d'alerte, ou
- un paramétrage de la fonction de traitement préliminaire, ou
- un réglage d'une temporisation de déclenchement d'alerte.

Selon une autre particularité, le réglage du module d'émission d'alerte comprend :
- un choix du type de message, ou
- un réglage du contenu du message, ou
- un réglage d'au moins une référence de variable d'alerte ou de séquence d'évènements informatiques insérée au contenu du message d'alerte envoyé, lors de l'activation du module d'émission d'alerte, ou
- un réglage de la destination du message, ou
- un réglage de paramètres de déblocage et de sortie d'un état d'alerte.

Selon une autre particularité, l'étape d'activation du module d'émission d'alerte est suivie d'au moins :
- une étape de désactivation du module d'évaluation,
- une étape de réglage automatique, par le module d'apprentissage, du module d'évaluation ou de son module de surveillance associé ou de son module d'émission d'alerte associé
- une étape d'activation du module d'évaluation.

Selon une autre particularité, l'étape de désactivation du module d'évaluation est précédée d'au moins :
- une étape d'envoi d'un message d'avertissement, vers l'interface utilisateur,
- une étape d'attente d'une entrée d'annulation de l'état d'urgence, par l'interface utilisateur,
- une étape de détection de l'entrée d'annulation suivie de l'étape de désactivation du module d'évaluation.

Selon une autre particularité, le module d'évaluation exécute au moins :
- une étape de temporisation avant validation de l'état d'urgence, débutant au dépassement, par la variable testée, d'un seuil paramétrable et s'interrompant dans le cas d'un retour de la variable testée sous le seuil.

Selon une autre particularité, l'activation du module d'émission d'alerte déclenche un appel téléphonique à l'aide d'un microphone, d'un haut-parleur ou d'une caméra.

Un autre objectif est de proposer un dispositif évolutif d'émission d'un ou plusieurs messages d'alerte en réponse à la détection d'un ou plusieurs évènements, permettant un paramétrage simple d'une ou plusieurs procédures d'urgence.

Cet objectif est atteint grâce à un dispositif portable en situation de mobilité associé à un réseau de télécommunication, comprenant au moins :
- des moyens de réception ou d'émission de données par le réseau,
- des moyens de traitement,
- des moyens de mémorisation,
caractérisé en ce qu'un contexte d'utilisation mémorisé comprenant des données représentatives d'au moins un état d'au moins un capteur, les moyens de traitement associés aux moyens de mémorisation sont agencés de façon à réaliser au moins :
un module d'analyse, initialisé en fonction du contexte d'utilisation et commandé dans un état actif ou dormant par un module de commande,
le module d'analyse comprenant au moins un module d'évaluation commandant au moins un module d'émission d'alerte initialisé en fonction du contexte, en fonction d'au moins une variable d'alerte réglée dans un état représentatif d'un capteur, par un module de surveillance initialisé en fonction du contexte,
au moins un module parmi le module d'analyse, le module d'émission d'alerte et le module de surveillance, étant réglé par l'utilisateur préliminairement à son activation,
le module d'analyse, dans son état actif, commandant par son module d'évaluation, l'activation du module d'émission d'alerte si la ou les variables testées sont représentatives d'un état d'urgence, le module d'émission d'alerte activé déclenchant l'envoi d'au moins un message d'alerte, par les moyens d'émission de données par le réseau ou par d'autres moyens d'émission.

Selon une autre particularité, le dispositif comprend un module de paramétrage réglant le module d'évaluation, de surveillance ou d'émission d'alerte, lorsque le module d'analyse est dans son état dormant, le module de paramétrage étant piloté par :
- une interface utilisateur, ou
- un module d'apprentissage, ou
- un module de commande à distance.

Selon une autre particularité, le module d'apprentissage dans son état actif mémorise des séquences d'évènements informatiques représentatives des états des modules de surveillance, d'évaluation et d'émission d'alerte, en concordance de phase avec les états des variables d'alerte.

Selon une autre particularité, un signal de commande à distance est reçu par le module de commande à distance qui pilote le module d'analyse à l'état actif ou dormant.

Selon une autre particularité, le dispositif comprend une bibliothèque de modules d'analyse, d'évaluation, de surveillance ou d'émission d'alerte dédiés chacun à une activité déterminée ou de modules d'analyse, d'évaluation, de surveillance ou d'émission d'alerte sauvegardés chacun avec des données associées d'initialisation représentatives du contexte d'utilisation lors de la sauvegarde.

Selon une autre particularité, le dispositif comprend au moins un capteur environnemental communiquant, à son module de surveillance associé, un signal représentatif d'une caractéristique physique ou chimique d'un environnement dans lequel se trouve le dispositif portable.

Selon une autre particularité, le dispositif comprend au moins un capteur biométrique communiquant, à son module de surveillance associé, un signal représentatif d'une caractéristique physiologique de l'utilisateur du dispositif portable.

Selon une autre particularité, le dispositif comprend au moins un capteur localisateur communiquant, à son module de surveillance associé, un signal représentatif d'une localisation géographique à laquelle se trouve le dispositif portable.

Selon une autre particularité, le dispositif comprend au moins un capteur de présence d'une carte personnelle communiquant, à son module de surveillance associé, un signal représentatif de la présence de la carte personnelle à proximité du dispositif portable.

Selon une autre particularité, le dispositif comprend au moins un module d'émission d'alerte supplémentaire, réalisant, dans son état activé, l'envoi d'un message de type et de contenu paramétrables déterminés, vers une destination paramétrable déterminée, par des moyens supplémentaires d'émission et de réception en liaison avec un réseau local ou le réseau Internet.

Selon une autre particularité, le module d'analyse comprend une pluralité de modules d'évaluation, correspondant chacun à un scénario d'évaluation et associés chacun à au moins un module de surveillance d'une variable d'alerte et à un module d'évaluation.

L'invention, ses caractéristiques et ses avantages apparaîtront plus clairement à la lecture de la description faite en référence aux figures ci-dessous référencées et données à titre d'exemples non limitatifs :
- la figure 1 représente un exemple de configuration d'un dispositif portable de communication selon l'invention ;
- la figure 2 représente un exemple de structure d'un dispositif portable de communication selon l'invention ;
- la figure 3 représente un exemple de procédé d'alerte selon l'invention ;
- la figure 4 représente un exemple de procédé de paramétrage d'un message d'alerte selon l'invention ;
- la figure 5 représente un exemple de procédé de paramétrage de surveillance d'un capteur selon l'invention ;
- la figure 6 représente un exemple de procédé de paramétrage d'un scénario d'alerte selon l'invention.

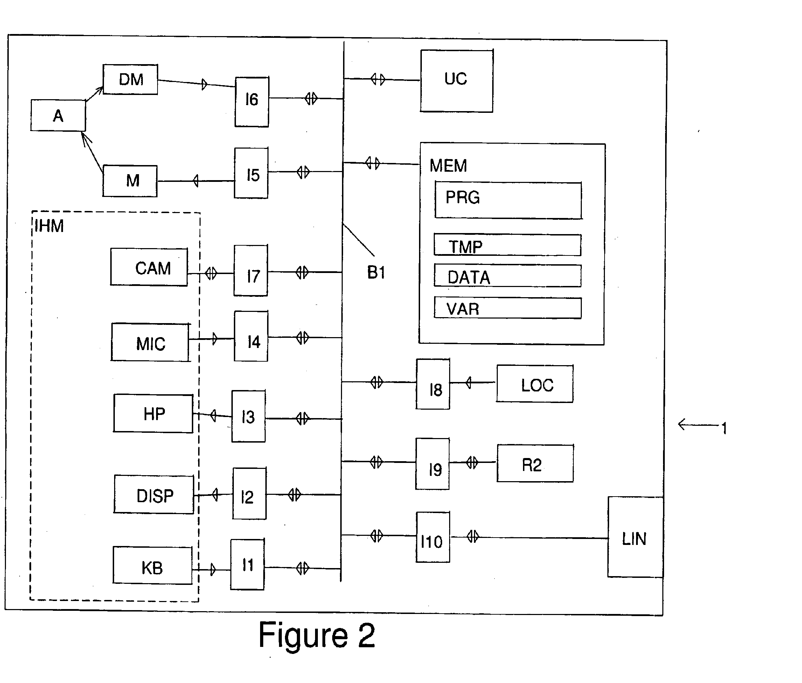

L'invention va à présent être décrite en référence aux figures précédemment citées. Un exemple d'architecture matérielle de l'appareil portable va maintenant être décrit. La figure 2 représente un exemple non limitatif d'un appareil (1) portable de télécommunication, par exemple, de type téléphone cellulaire, ordinateur personnel ou appareil électronique embarqué. L'appareil de télécommunication comprend, par exemple, une unité de calcul (UC) en communication via un bus (B1) de données, avec une mémoire (MEM) et avec plusieurs interfaces (I1, I2, I3, I4, I5, I6) de communication. Différents éléments constituent de manière non limitative, une interface (IHM) de communication avec l'utilisateur : un appareil (1) portable de communication comprend par exemple un microphone (MIC) d'enregistrement de la voix de l'utilisateur, un haut parleur (HP), un écran (DISP) d'affichage et un clavier (KB). Chacun des éléments (HP, MIC, DISP et respectivement KB) de l'interface utilisateur est, de manière non limitative, en communication avec le bus (B1) de données, via une interface (I3, I4, I2 et respectivement I1) de communication. Chacun des composants de l'appareil (1), à l'exception de l'unité de calcul, est contrôlé par un dispositif de contrôle non représenté ou par l'unité de calcul. L'unité (UC) de calcul, par exemple un circuit intégré de type ASIC ou composant programmable de type FPGA ou un microprocesseur, traite des données sous forme numérique. Les moyens de traitement comprennent, par exemple, l'unité de calcul.

De manière non limitative, le dispositif de communication, supportant par exemple la visioconférence, est équipé d'une caméra (CAM) en communication, par son interface (I7), avec un bus (B1) de données et de contrôle. La caméra (CAM) est par exemple commandée par l'unité (UC) de calcul. Des données représentatives d'images capturées par la caméra sont par exemple, stockées dans un espace (TMP) mémoire déterminé, pour être traitées par les moyens de traitement.

L'appareil portable de communication comprend un composant (M) de modulation et un composant (DM) de démodulation. Le composant (M) de modulation et le composant (DM) de démodulation communiquent avec le bus (B1) de données, via leur interface (I5, I6) de communication. Les composants (M, DM) de modulation et de démodulation sont reliés à une antenne (A) d'émission et de réception d'ondes radio. Lors d'une émission, le composant (M) de modulation transforme une information numérique, provenant du bus (B1) de données, en une information analogique, sous la forme d'un signal électrique. Ce signal électrique est transformé en ondes radio représentatives de ce signal, par l'antenne.

Lors d'une réception, l'antenne (A) transforme, par exemple, les ondes radio reçues en un signal électrique analogique. Le composant (DM) de démodulation transforme le signal analogique représentatif des signaux radio en un signal numérique représentatif envoyé sur le bus (B1) de données, via l'interface (I6) associée au composant (DM) de démodulation. L'information, de type numérique, en entrée du composant (M) de modulation ou en sortie du composant (DM) de démodulation comprend différentes données dépendantes, de manière non limitative, du protocole de communication et de l'information transmise. Le dispositif portable de communication peut ainsi recevoir tout type d'information, comme par exemple des informations représentatives d'un module logiciel ajouté au dispositif portable. De manière non limitative la communication par les ondes radio est réalisée selon le protocole GSM (Global System for Mobile Communications), GPRS (Global Packet Radio Service), UMTS (Universal Mobile Telecommunications System) ou HSDPA (High Speed Downlink Packet Access). Le type de modulation est, par exemple, du type W-CDMA (Wideband Code Division Multiple Access) comme pour le protocole UMTS ou de type AMRT (Accès Multiple à Répartition dans le Temps) combiné à AMRF (Accès Multiple à Répartition de Fréquence) comme pour le protocole GSM.

De manière non limitative, un connecteur (LIN) communique, par une interface (I10) avec le bus (B1) du dispositif de communication. L'interface (I10) du connecteur est par exemple, réalisée pour transmettre ou recevoir des données numériques ou analogiques selon un protocole de communication déterminé. Le connecteur (LIN) permet, par exemple, de brancher un capteur ou un dispositif de contrôle ou de mesure, transmettant des signaux numériques ou analogiques à l'interface (I10) associée. Cette interface transmet, d'autre part, des données numériques représentatives des signaux reçus, à l'unité de calcul (UC), pour être traitées ou stockées en mémoire (MEM).

Selon un exemple non limitatif, le capteur est un capteur biométrique transmettant des signaux représentatifs d'un paramètre biométrique mesuré, comme par exemple, le rythme cardiaque ou d'autres caractéristiques biologiques de l'utilisateur.

Selon un exemple non limitatif, le capteur relié au connecteur (LIN) est un capteur de température ou un capteur d'humidité ou un analyseur chimique ou un autre capteur, transmettant des signaux représentatifs de caractéristiques environnementales.

Selon un exemple non limitatif, le capteur connecté au dispositif de communication est un capteur de position transmettant des signaux représentatifs de la position du buste ou de la tête de l'utilisateur, utilisé, par exemple, pour la détection d'une chute de l'utilisateur.

De manière non limitative, le dispositif (1) de communication comprend un dispositif de localisation (LOC) géographique, communiquant, via son interface associée (I8), avec le bus (B1) en communication avec l'unité (UC) de calcul et la mémoire (MEM). Le dispositif de localisation (LOC) transmet, par exemple, des données représentatives de la position géographique du dispositif (1) de communication. Le dispositif de localisation est, par exemple, un dispositif de type GPS (Global Positioning System) qui permet, en liaison avec un «système satellite», de déterminer une localisation géographique. Cette localisation géographique correspond, par exemple, à la longitude et la latitude du dispositif (1) de communication ou correspond à une adresse postale ou une localisation sur un plan de ville ou une carte routière. Le composant GPS produit, par exemple, des informations de localisation géographique sous forme numérique. De manière non limitative, les informations de localisation géographique sont placées dans une zone mémoire (TMP) ou sont traitées par l'unité de calcul.

De manière non limitative, le dispositif de communication (1) comprend un second circuit (R2) d'émission et de réception, pour établir une communication par ondes radiofréquences, comme par exemple Wi-Fi ou Bluetooth®, ou par liaison optique. Le second circuit d'émission et de réception comprend, par exemple, un émetteur et un récepteur radiofréquence ou infrarouge. De façon avantageuse ce second (R2) circuit d'émission et de réception permettra d'acquérir tout type d'information, par exemple, par l'intermédiaire d'ordinateur de bureau ou d'autres machines. Un utilisateur, comme par exemple un particulier ou un professionnel, pourra ainsi charger, par ses propres machines, un module informatique interagissant dans son dispositif portable de communication.

Le second circuit (R2) d'émission et de réception communique, via une interface (I9) associée, avec le bus (B1) qui communique avec l'unité (UC) de calcul et la mémoire. L'interface (I9) réalise par exemple, une conversion de données numériques provenant du bus, en données analogiques transmises au second circuit (R2) d'émission et de réception, pour être transformées en ondes radiofréquences ou en ondes lumineuses. Les ondes radiofréquences ou lumineuses reçues, par exemple, par le second circuit (R2) d'émission et de réception, sont transformées en signaux électriques analogiques, convertis, par l'interface associée en données numériques. D'autre part l'unité (UC) de calcul exécute, par exemple, un programme d'interface déterminé pour établir la communication, par exemple, selon le protocole Wi-Fi ou Bluetooth®. Le dispositif peut ainsi être connecté directement au réseau Internet ou à un réseau local, par exemple, pour transmettre un e-mail ou adresser une requête à un serveur déterminé.

L'architecture du dispositif portable de communication en situation de mobilité ne se limite pas bien entendu aux exemples précédemment cités. Ainsi le dispositif portable de communication peut être disposé dans un véhicule, tel qu'une automobile, comprenant ses propres capteurs reliés au dispositif portable de communication. Le dispositif portable de communication peut aussi passer d'un type de réseau à un autre, pour établir une communication. La détection d'une communication établie par un réseau déterminé, permet par exemple, de régler des modules logiciels actifs en correspondance avec les modes de communication de ce réseau.

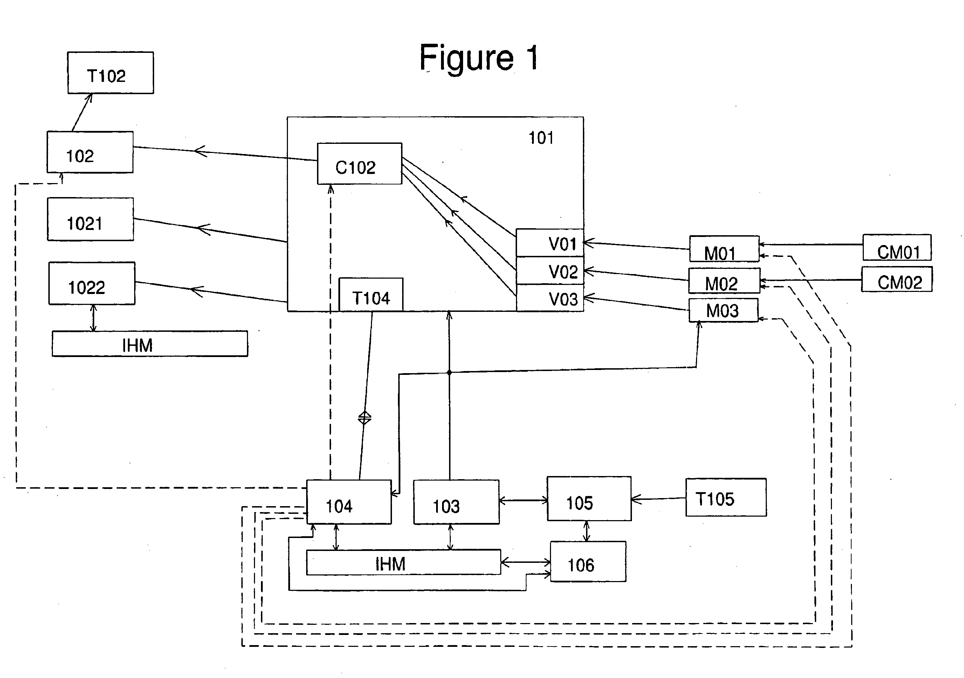

Un exemple de configuration du dispositif portable de communication va maintenant être décrit. De manière non limitative, la mémoire (MEM) est divisée en plusieurs zones comme par exemple, une zone (PRG) réservée aux programmes, une zone (DATA) de stockage de données, une zone (VAR) réservée pour des variables et une zone (TMP) tampon ou de stockage temporaire. Le dispositif de communication comprend, par exemple, plusieurs modules logiciels activés en même temps et communiquant entre eux, comme représenté à la figure 1. Des programmes sont exécutés lors de l'activation des modules logiciels qui réalisent, par exemple, des lectures ou des écritures dans les différentes zones mémoires ou directement dans les différentes interfaces (I1, I2, I3, I4, I5, I6, I7, I8, I9, I10) communiquant avec le bus (B1). Un module logiciel comprend, par exemple, des variables, des paramètres, des données et des méthodes ou des programmes.

Le dispositif de communication comprend, par exemple, un ou plusieurs modules (101) d'analyse. Une bibliothèque de modules d'analyse, stockée en mémoire, pourra, par exemple, être enrichie par des modules d'analyse propres à un usage déterminé, comme par exemple des modules dédiés au tourisme ou au journalisme permettant une assistance sur place de l'utilisateur. Un module d'analyse sera, par exemple, structuré autour d'un ou plusieurs programmes de calcul et d'évaluation recevant des données d'entrée et fournissant des données de sortie. Des programmes d'évaluation pourront s'exécuter de façon autonome ou interagir entre eux. De manière non limitative, un module logiciel sera initialisé par des paramètres d'initialisation représentatifs d'un nom du module ou d'un contexte d'utilisation du terminal. Le contexte d'utilisation du terminal sera, par exemple, représenté par un ensemble des données produites par les capteurs à un instant déterminé. Le nombre et le type des capteurs sont, par exemple, pris en compte lors de l'initialisation. Un état d'un ou plusieurs capteurs peut aussi être pris en compte.

Pour analyser une situation selon un ou plusieurs critères, un module (101) d'analyse communique, par exemple, avec un ou plusieurs modules (M01, M02, M03) de surveillance. Un module (M01, M02, M03) de surveillance règle, par exemple, une variable (V01, V02, V03) d'alerte du module d'analyse. Un ou plusieurs modules (M01, M02, M03) de surveillance règlent, par exemple, une ou plusieurs variables (V01, V02, V03) d'alerte associées, chacune à un état représentatif ou non d'un état d'urgence. La variable d'alerte est, par exemple, une variable binaire ou une variable représentative d'un entier ou d'un réel ou un tableau d'entiers ou de réels. Ces variables (V01, V02, V03) positionnées par les modules (M01, M02, M03) de surveillance, sont par exemple, utilisées comme entrée d'une fonction d'évaluation d'un niveau d'alerte. De manière non limitative, un module (M01, M02, M03) de surveillance communique avec un ou plusieurs modules (101) d'analyse. Une bibliothèque de modules de surveillance, stockée en mémoire, pourra, par exempte, être enrichie par des modules de surveillance propres à un usage déterminé. Un capteur ou un détecteur particulier, nécessitera par exemple, un module de surveillance associé permettant une interprétation des signaux ou des données fournies par ce capteur. Un capteur est par exemple, associé, en sortie, à un filtrage et une amplification déterminés. Un module de surveillance est, par exemple, associé à plusieurs capteurs déterminés, le module de surveillance réalisant des pondérations des données provenant des différents capteurs, ces pondérations pouvant être répétées ou conditionnelles. De manière non limitative, ces pondérations sont paramétrables.

Un module (M01) de surveillance est, par exemple, en communication avec un capteur (CM01) et lie ou traite des données numériques représentatives de l'état du capteur (CM01) pour régler une variable de sortie, comme par exemple, une variable (V01) d'alerte. De manière non limitative, le module de surveillance réalise un accès à une mémoire tampon (TMP) dans laquelle des données représentatives de l'état du capteur sont inscrites ou le module de surveillance accède à l'interface de communication du capteur. Un module de surveillance réalisera ainsi un traitement préliminaire des données correspondant à l'état du capteur. Ce traitement préliminaire est, par exemple, une mise à l'échelle, une pondération ou une comparaison à un seuil.

Selon un exemple non limitatif, un module (M03) de surveillance réalise un comptage à une fréquence déterminée et produit un signal de sortie représentatif de l'écoulement d'une temporisation. De manière non limitative, la temporisation débute lors de l'activation du module d'analyse (101) associé, ou est déclenchée selon l'état d'un capteur.

Pour analyser finement une situation selon un ou plusieurs scénarii, le module (101) d'analyse comprend, par exemple, un ou plusieurs modules (C102) d'évaluation paramétrables. Un module (C102) d'évaluation comprend, par exemple, une fonction ou un programme d'évaluation dont la ou les entrées sont, par exemple, des variables (V01, V02, V03) d'alerte accédées par des pointeurs du module (C102) d'évaluation. Le module (C102) d'évaluation traite ainsi les variables (V01, V02, V03) d'alerte selon un scénario déterminé, en fonction des variables d'alerte, pour déclencher, en cas d'urgence, l'envoi d'un ou plusieurs messages d'alerte. Différents niveaux d'urgence correspondant à différents seuils ou différentes situations d'urgence sont programmables pour déclencher des procédures d'urgence associées. Le module (101) d'analyse est, par exemple, associé à un ou plusieurs modules (102, 1021, 1022) d'alerte commandés chacun dans un état désactivé ou activé. Un module (C102) d'évaluation du module d'analyse commande, par exemple, un module (102) d'alerte associé, dans un état désactivé, par défaut, et dans un état activé, lorsque son programme d'évaluation détecte un état d'urgence, en fonction de ses variables d'alerte associées. Une bibliothèque de module d'évaluation ou une bibliothèque de modules d'alerte, stockée en mémoire, pourra être enrichie par des modules spécialement adaptés à une activité déterminée.

De façon avantageuse, une bibliothèque pourra être enrichie par des modules, dits « métier », adaptés à une activité déterminée et les modules logiciels, après avoir été initialisés, pourront être réglés automatiquement par un module (106) d'apprentissage ou manuellement par l'utilisateur ou à distance par le module (105) de commande à distance. La sauvegarde des modules réglés, correspondant à un contexte d'utilisation déterminé pour un usage déterminé, permet d'enrichir encore la bibliothèque.

Un module (102) d'alerte est, par exemple, associé à un module (T102) d'émission pour envoyer un message d'alerte, lorsque le module d'alerte est activé. Le module (102) d'alerte est ainsi associé à un type de message compatible avec son module (T102) d'émission. De manière non limitative, le module (102) d'alerte comprend un message déterminé dont le contenu est paramétré à l'avance ou enrichi au moment de l'activation de l'alerte, pour être envoyé vers une ou plusieurs destinations paramétrées et mémorisées dans le module d'alerte.

Ainsi des variables (V01, V02), par exemple, représentatives de l'état de capteurs (CM01, CM02) en communication avec le module (101) d'analyse, via des modules (M01, M02) de surveillance, sont analysées par un module (C102) d'évaluation. Les capteurs (CM01, CM02) de déclenchement de l'alerte sont, par exemple, des capteurs biométriques se mettant dans un état d'alerte si les paramètres relevés sont anormaux ou témoignent d'un état de faiblesse de l'utilisateur. Le module (C102) d'évaluation active, par exemple, un module (102) d'alerte, si l'exécution de son programme d'évaluation détermine que les variables testées sont représentatives d'un état d'urgence. Le module (102) d'alerte activé réalise, par exemple, l'envoi d'un message d'urgence, de type texte, adressé à un bureau de surveillance ou à un service hospitalier, contenant un identifiant de l'utilisateur et la position géographique relevée par un dispositif de localisation de type GPS (Global Positioning System).

Le module (101) d'analyse, n'est pas actif en permanence, mais est activé, par exemple, dans une situation potentiellement risquée. De manière non limitative, un module (101) d'analyse est activé par l'utilisateur via une interface homme machine ou est activé à distance par un module (105) en communication, par un réseau de télécommunication, avec un serveur de gestion de la sécurité de l'utilisateur.

De manière non limitative, un module (103) de commande, piloté par une interface (IHM) utilisateur, par le module (105) de commande à distance ou par le module (106) d'apprentissage, commande, par exemple, le module (101) d'analyse, dans un état activé ou dormant. Un module d'analyse associé à des modules de surveillance de paramètres biométriques est, par exemple, activé par un utilisateur devant parcourir seul une région escarpée où un accident pourrait survenir. D'autre part, lorsque ce module d'analyse est désactivé, l'utilisateur peut débrancher les capteurs biométriques.

De manière non limitative, un module d'analyse peut être, par exemple, activé par un enfant lors d'un trajet entre son école et son logement. L'enfant accède, par exemple, par un menu affiché à l'écran (DISP) à la commande d'activation du module d'analyse. Ce module d'analyse est, par exemple, associé à un bouton d'alerte activé en cas de danger ou à un chronomètre déclenchant une alerte si le temps de parcours dépasse une durée maximum déterminée. Un message texte, de type SMS, est par exemple, envoyé aux parents de l'enfant, si l'alerte est déclenchée, afin de permettre aux parents de réagir immédiatement.

De manière non limitative, le module (105) de commande à distance reçoit, par exemple, une commande d'activation d'un module d'analyse déterminé par un module (T105) de réception des messages de commande. Ce module (T105) de réception des messages est par exemple un module logiciel associé à l'interface (16) de réception de données radiofréquence. Ainsi un ou plusieurs scénarii peuvent être activés automatiquement, à la réception d'un message de commande d'activation de surveillance. Ce message d'activation de surveillance est, par exemple, transmis par une société à un employé exerçant un métier à risque.

Pour adapter le dispositif de communication d'alerte à des situations nombreuses et variées, lorsqu'un module (101) d'analyse est dans son état dormant, un module (104) de paramétrage permet de régler de nombreux paramètres concernant, par exemple, le module (101) d'analyse ou les modules (M01, M02, M03) de surveillance associés ou les modules (102) d'alerte associés. De nombreux capteurs peuvent ainsi être sélectionnés pour que les données, représentatives de leurs états, soient interprétées selon plusieurs scénarii possibles du module d'analyse, afin de déclencher plusieurs alertes possibles. Un ou plusieurs modules d'analyse permettent ainsi d'interpréter une situation déterminée.

Pour perfectionner encore le dispositif d'alerte en situation de mobilité, le module (106) d'apprentissage peut, de manière non limitative, paramétrer ou régler automatiquement les modules de surveillance, d'analyse et d'alerte, par exemple via le module (104) de paramétrage. Le module (106) d'apprentissage réalise, par exemple, un enregistrement périodique des variables (V01, V02, V03) d'alertes en concordance de phase avec des données représentatives de l'état des modules de surveillance, d'analyse et d'alerte associés. De cette façon le module d'apprentissage capitalise de l'expérience sous la forme de séquences d'évènements informatiques. De plus, l'ensemble des variables est pris en compte quels que soient les modules de surveillance actifs. De manière non limitative, le module d'apprentissage est associé à une zone mémoire de stockage des séquences d'évènements informatiques sur lesquelles un ou plusieurs programmes d'examen sont appliqués. Les programmes d'examen appliqués aux séquences d'évènements informatiques sont, par exemple, importés et structurés pour réaliser une analyse des réactions de l'utilisateur. Le comportement de l'utilisateur permet ainsi d'adapter l'analyse de surveillance et d'alerte au cas par cas. De manière non limitative, le module (106) d'apprentissage comprend ses propres programmes d'adaptation déterminant les réglages des modules.

De manière non limitative, la fonction de réglage automatique, par le module (106) d'apprentissage, des modules d'analyse, d'évaluation, de surveillance ou d'émission d'alerte, peut être désactivée ou activée par l'interface (IHM) utilisateur ou par le module (105) de commande à distance.

Selon un autre exemple, les réglages à effectuer par le module d'apprentissage résultent d'un dialogue avec un système expert extérieur. Un système central d'examen des séquences d'évènements informatiques permettra de plus de capitaliser l'expérience d'une flotte de dispositifs portables et de régler chaque dispositif portable, en conséquence.

Le module (106) d'apprentissage réalisera, par exemple, une initialisation des modules informatiques ou de leurs paramètres, lorsque ces modules d'analyse sont désactivés.

De manière non limitative, la fonction d'examen du module (106) d'apprentissage est activé par l'utilisateur, via l'interface (IHM) utilisateur, ou par le module (105) de commande à distance ou automatiquement de façon périodique.

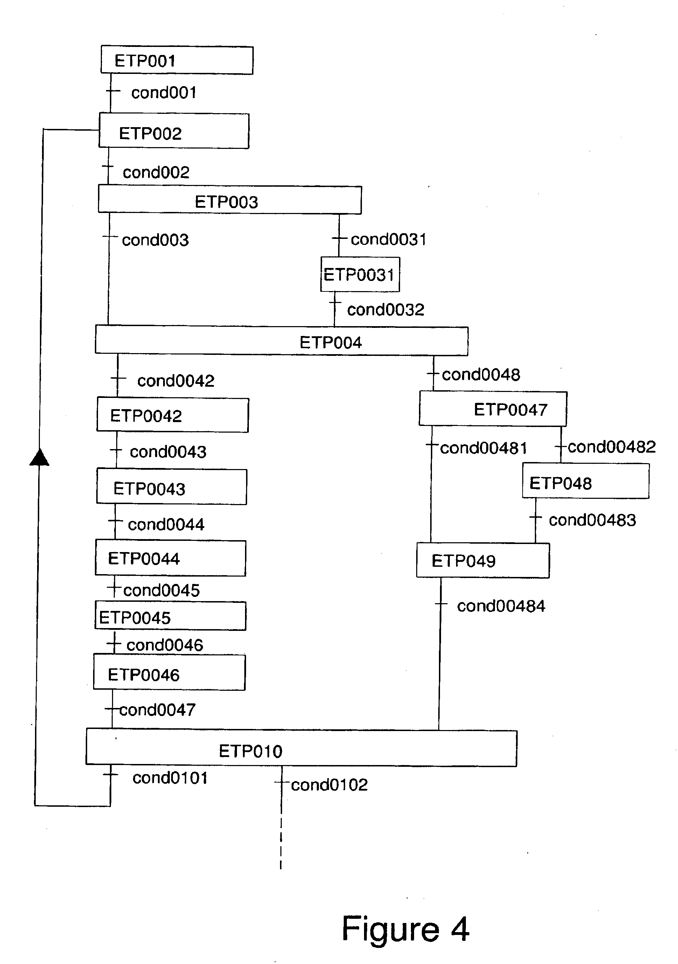

Un exemple de paramétrage d'un module d'émission d'alerte va maintenant être décrit. La figure 4 représente un exemple non limitatif de procédé de paramétrage d'une alerte à envoyer dans une situation d'urgence. L'utilisateur réalise, par exemple, une demande (ETP001) de paramétrage d'un module (101) d'analyse déterminé, via une interface (IHM) homme machine. Le module d'analyse regroupe par exemple, plusieurs programmes d'évaluation permettant des évaluations selon plusieurs scénarii d'alerte. Le module (104) de paramétrage reçoit et interprète, par exemple, des données de l'interface utilisateur entrées par l'utilisateur. L'interface homme machine comprend, par exemple, l'écran (DISP) sur lequel est affiché un menu proposant différentes actions sélectionnées grâce au clavier (KB). De manière non limitative, les menus proposés à l'utilisateur comprennent chacun des choix multiples dont un retour au menu précédent.

Si le module d'analyse est dans un état dormant (cond001), l'interface (IHM) utilisateur propose (ETP002), par exemple, un choix du type de paramétrage entre le paramétrage des déclencheurs ou le paramétrage des scénarii d'analyses ou le paramétrage des alarmes. Le module (104) de paramétrage pointant sur le module (101) d'analyse, accède, par exemple, à un tableau (T104) du module (101) d'analyse comprenant, pour chacun des modules associés au module (101) d'analyse, un point d'accès et le type du module.

Si l'utilisateur choisit, via l'interface (IHM) homme machine, le paramétrage des alertes (cond002), le module (104) de paramétrage propose, par exemple, de sélectionner une alerte (ETP003). De manière non limitative, le module (104) de paramétrage commande l'affichage de l'écran (DISP) via son interface (I2) pour afficher un choix parmi plusieurs alertes. L'actionnement du clavier (KB) est interprété, par le module (104) de paramétrage, pour signifier à l'utilisateur son choix en cours ou pour valider ce choix. L'utilisateur peut, par exemple, choisir de sélectionner une des alertes existantes ou de créer une nouvelle alerte.

Si l'utilisateur sélectionne une des alertes existantes (cond003), le module (104) de paramétrage sélectionne (EPT004), par exemple, le module (102) d'alerte choisi, pour le modifier ou le supprimer.

Dans le cas où l'utilisateur entre, via l'interface de pilotage, le choix d'une nouvelle alerte (cond0031), la création (ETP0031) d'un nouveau module d'alerte associé au module (101) d'analyse, est réalisée. Le module créé est, par exemple, un module d'alerte par défaut ou un module d'alerte standard choisi dans une bibliothèque de la mémoire (DATA) ou encore par un module d'alerte complètement vierge. De manière non limitative, des données représentatives du contexte sont utilisées pour l'initialisation du nouveau module d'alerte.

Après l'enregistrement (cond0032) de ce nouveau module d'alerte, le nouveau module est sélectionné à l'étape suivante, pour que l'utilisateur commande une modification ou une suppression du module. En cas d'erreur, le nouveau module est, par exemple, immédiatement supprimé. Si l'utilisateur a choisi un nouveau module comprenant des paramètres par défaut, déjà initialisés, l'utilisateur a alors la possibilité de revenir aux menus précédents, par la touche de retour au menu précédent. Le module (104) de paramétrage crée par exemple, un module logiciel d'alerte dont les points d'entrée et le type sont par exemple, mémorisés dans le tableau (T104) du module d'analyse (101).

A l'étape (ETP004) de configuration de l'alerte sélectionnée, l'utilisateur choisit, par exemple, de paramétrer cette alerte (cond0042) ou de supprimer (cond0048) cette alerte.

Si l'utilisateur choisit de paramétrer cette alerte, le module (104) de paramétrage modifie, par exemple, le module (102) d'alerte sélectionné. Une première étape (ETP0042) de configuration de l'alerte comprend par exemple, la configuration du type de message d'alerte. L'utilisateur choisit, par exemple, un type de message associé à un moyen d'envoi.

De manière non limitative, le menu de configuration de l'alerte propose l'utilisation des moyens de communication radio, par le réseau cellulaire, pour la transmission de messages du type texte, photo, film ou message vocal. Les messages d'alerte sont par exemple, des messages de type SMS, ou MMS. Le module (104) de paramétrage commande, par exemple, le module d'alerte pour mémoriser une donnée représentative du type de message ainsi qu'un pointeur vers l'interface (I5) associée au composant de modulation relié à l'antenne radio de connexion au réseau cellulaire.

De manière non limitative, un module (102) d'alerte peut utiliser le réseau téléphonique cellulaire pour envoyer un appel entrant vers un numéro déterminé. Le type d'appel est, de manière non limitative, un appel audio ou un appel vidéo utilisant, par exemple le standard UMTS.

De manière non limitative, le menu de configuration de l'alerte propose l'utilisation des moyens (R2) supplémentaires de communication radio, par exemple, conforme à la norme Wi-Fi ou Bluetooth® ou conforme à un autre standard pour établir une communication radio. Le type de message proposé est par exemple un message de type courrier électronique ou une requête standard envoyée vers un serveur d'un site Internet ou sur un réseau local. Le module (104) de paramétrage commande, par exemple, le module (102) d'alerte pour mémoriser le type de message ainsi qu'un pointeur vers l'interface (I5) associée au composant supplémentaire d'émission et de réception radio.

De manière non limitative, l'alerte peut être transmise ou enregistrée par des composants de l'interface (IHM) homme machine comme par exemple, le haut parleur (HP), l'écran (DISP), le microphone (MIC) ou le clavier (KB) ou la caméra (CAM). Le type de message proposé est, par exemple, un message de type texte, image, message sonore ou vidéo, transmis à l'interface utilisateur. Un message sonore est par exemple, répété en boucle, avec un volume maximum, pour prévenir d'une faiblesse physique détectée par un capteur biométrique. De façon avantageuse un capteur biométrique permet de contrôler un paramètre relatif à la santé de l'utilisateur et permet aussi d'enregistrer des réactions de l'utilisateur.

Selon un autre exemple un message sonore est par exemple répété en boucle pour prévenir d'un vol de portable détecté. Le module d'alerte peut afficher une invitation à entrer un code secret, le téléphone restant par exemple, bloqué dans cet état d'alerte tant qu'un code mémorisé dans le module d'alerte n'a pas été entré.

Après l'enregistrement (cond0043) du type de message associé au moyen de communication utilisé, dans le module d'alerte, le module de paramétrage réalise une étape (ETP043) de définition du contenu.

Selon un exemple non limitatif, le contenu du message est défini à partir d'une bibliothèque stockée dans un espace (DATA) mémoire. La bibliothèque sera, par exemple, enrichie par des contenus, dits « métier », spécifiques à une activité professionnelle. Le module de paramétrage comprend, par exemple, un pointeur vers cette bibliothèque pour afficher certains de ses éléments à l'écran, pour que l'utilisateur réalise une sélection parmi les éléments affichés. Le module (104) de paramétrage n'affiche, par exemple, que les éléments correspondant au type d'alerte enregistré. De manière non limitative, les messages prédéfinis sont de type texte, photo, vidéo ou son. Selon un autre exemple, le contenu du message d'alerte correspond à une requête destinée à un serveur déterminé ou un message de commande pour un appareil déterminé.

Selon un exemple non limitatif, l'étape (ETP0043) de définition du contenu réalisée par le module (104) de paramétrage, comprend l'entrée, par l'interface utilisateur, de données représentatives de texte ou de sons ou respectivement d'images par le clavier (KB) ou le micro (MIC) ou respectivement la caméra (CAM).

Après enregistrement du contenu (cond0044) une étape (ETP0044) de choix d'insertion d'informations instantanées est réalisée, de manière non limitative, par le module (104) de paramétrage. L'utilisateur définit par exemple, via l'interface (IHM) homme machine, un ensemble de données insérées dans le contenu du message d'alerte avant son envoi.

Selon un exemple non limitatif, des données représentatives d'informations instantanées capturées lors du déclenchement de l'alerte, sont incorporées au message. Le module (104) de paramétrage configure par exemple, la capture à réaliser, en mémorisant, par exemple, une adresse à laquelle ces données instantanées sont mémorisées. Les données instantanées sont, par exemple, les dernières coordonnées géographiques produites par le dispositif de localisation géographique. Selon un autre exemple les données instantanées correspondent à un enregistrement sonore d'une durée déterminée, réalisé par le microphone. Selon un autre exemple, les données instantanées correspondent à l'état d'un capteur, ces données étant par exemple, stockées continuellement dans une zone mémoire, avec un écrasement périodique des anciennes données. Ainsi le module d'alerte lira à une adresse déterminée, les données instantanées mémorisées.

Le module (104) de paramétrage enregistre, par exemple, dans le module (102) d'alerte sélectionné, une référence pointant sur une zone mémoire déterminée. La zone mémoire est par exemple une zone (TMP) tampon dans laquelle des données représentatives des images capturées par la caméra sont enregistrées. Ainsi le récepteur du message d'alerte pourra visionner des images donnant un aperçu de l'environnement de l'utilisateur aux instants voisins du déclenchement de l'alerte. Un utilisateur portant un dispositif de communication dans une poche, à hauteur de son épaule, peut, par exemple, transmettre des images de personnes qui ont agressé l'utilisateur, les agresseurs étant par exemple, en cause dans le déclenchement de l'alerte.

Selon un exemple non limitatif, des données représentatives de la position géographique du dispositif de communication, sont insérées dans le contenu du message à transmettre. Le module (104) de paramétrage enrichit, par exemple, le contenu du message, en enregistrant dans le module d'alerte, une référence pointant sur une zone tampon (TMP) dans laquelle les données produites par le dispositif de localisation sont stockées. Ainsi le récepteur du message d'alerte pourra connaître les positions successives du dispositif de communication avant ou pendant l'état d'urgence.

Selon un exemple non limitatif, le déclenchement d'un état d'urgence, bloque le téléphone dans un état déterminé dans lequel des messages d'alerte sont envoyés en continu, pour indiquer les positions géographiques successives du dispositif de communication ou des caractéristiques environnementales ou biométriques successives.

Selon un exemple non limitatif, le module (104) de paramétrage enregistre la référence d'une zone mémoire tampon (TMP) recevant des données produites par un capteur biométrique, permettant de surveiller à distance et en continu l'état du patient.

Selon un exemple non limitatif, l'enrichissement du message d'alerte est réalisé par l'insertion de données représentatives d'une pluralité d'informations. Le module (104) de paramétrage enregistre, par exemple, les références de plusieurs zones mémoire de stockage correspondant à plusieurs capteurs ou à plusieurs modules de surveillance.

Selon un exemple non limitatif, après l'enregistrement (cond0045), dans le module (102) d'alerte, de références des données instantanées, le module (104) de paramétrage exécute une étape (ETP0045) de définition d'une destination. Le module (104) de paramétrage propose, par exemple par un menu, un ensemble de destinations provenant d'un répertoire ou l'entrée de données représentatives d'une nouvelle destination est proposée à l'utilisateur. Une ou plusieurs destinations sont associées à un message d'alerte. Une destination est, par exemple, un numéro de téléphone ou un numéro de fax ou une adresse postale électronique ou l'adresse d'un serveur recevant des requêtes d'alerte. La destination d'un message d'alerte peut aussi être le haut parleur (HP) recevant un message sonore ou l'écran (DISP) affichant un message d'alerte ou encore le connecteur (LIN) auquel un dispositif de diffusion du message d'alerte est connecté.

Selon un exemple non limitatif, la destination est déterminée par défaut ou la destination est unique, comme par exemple, pour un message sonore d'alerte à diffuser en continu par le haut parleur (HP), jusqu'au déblocage de l'état d'alerte. Ainsi dans le cas où le vol du dispositif est détecté, le dispositif peut se placer dans un état de blocage de toutes les fonctions avec un message affiché à l'écran, tandis qu'un message sonore indiquant le vol, est diffusé en continu. D'une part ce type d'alerte est dissuasif mais le message sonore avertit aussi l'utilisateur d'un vol ou d'un danger.

Selon un exemple non limitatif, après l'enregistrement de la ou des destinations du message d'alerte (cond0046), le module (104) de paramétrage exécute une étape (ETP0046) de définition des paramètres de déblocage de l'état d'urgence. Ces paramètres sont, par exemple, un code de déverrouillage. Un état d'urgence correspond, par exemple, à un état dans lequel un module (102) d'alerte est activé par le module (101) d'analyse. L'utilisateur peut, de manière non limitative, désactiver l'état d'alerte en entrant son code personnel d'identification, qui peut être tout simplement le code PIN, ou en entrant un autre code secret.

Le module (104) de paramétrage enregistre, par exemple, dans le module (102) d'alerte les références de variables mémorisées dans une zone mémoire (MEM) pour être comparées avec des données rentrées via l'interface homme machine. Les variables mémorisées correspondent, par exemple, à une combinaison de touches ou à des touches activées successivement ou à un mot ou une phrase reconnu par reconnaissance vocale ou encore à un paramètre biométrique. Ainsi le dispositif (1) portable de communication peut mémoriser l'empreinte vocale de l'utilisateur et autoriser le déblocage de l'état d'alerte lorsque cette empreinte est reconnue.

Selon un exemple non limitatif, le module (104) de paramétrage réalise un enregistrement d'un mot clé prononcé par la personne pouvant débloquer le dispositif. Des données représentatives de ce mot clé sont, par exemple, stockées en mémoire (MEM) et les références de ces données sont enregistrées dans le module (102) d'alerte. Ainsi le module d'alerte bloqué dans un état activé, sera débloqué par la prononciation du mot clé, par l'utilisateur. Le module d'alerte réalise, par exemple, une comparaison des données représentatives des sons enregistrés par le micro avec les données de déblocage, pour passer ou non dans un état désactivé.

Selon un exemple non limitatif, le module d'alerte après l'envoi d'un ou plusieurs messages d'alerte, retourne automatiquement dans sa position non activée.

Après l'enregistrement (cond0047) du ou des paramètres de déblocage, le module (104) de paramétrage propose, par exemple, une étape (ETP010) de choix de sortie ou non du mode de paramétrage.

Un exemple de suppression d'un module d'alerte va maintenant être décrit. L'utilisateur choisit, par exemple, via l'interface (IHM) homme machine, à l'étape (ETP004) de configuration de l'alerte sélectionnée, la suppression (cond0048) de l'alerte sélectionnée.

De manière non limitative, le module (104) de paramétrage propose (ETP0047) une sauvegarde de l'alerte sélectionnée, avant sa suppression. Si l'utilisateur choisit, via l'interface utilisateur, de sauvegarder cette alerte (cond00482), le module (102) d'alerte sélectionné est, par exemple, mémorisé (ETP048) dans un espace (DATA) mémoire de stockage d'une bibliothèque d'alerte comprenant des alertes par défaut et éventuellement des alertes paramétrées par l'utilisateur. La bibliothèque de modules d'alerte peut ainsi être enrichie par des modules configurés au cas par cas. Le module (102) d'alerte nouvellement stocké est par exemple, associé à un nom d'alerte, entré par l'utilisateur, ou à un contexte du dispositif portable de communication, utilisés, par exemple lors de l'initialisation.

A la fin de la sauvegarde (cond00483) ou après le choix (cond00481) de ne pas réaliser de sauvegarde, le module (102) d'alerte est par exemple supprimé (ETP0049). Après suppression du module d'alerte (cond00484) le module (104) de paramétrage propose (ETP010) soit de continuer (cond0101) le paramétrage, soit d'arrêter (cond0102) le paramétrage.

L'utilisateur peut, par exemple, choisir de continuer le paramétrage du module (101) d'analyse, le module (104) de paramétrage réalisant, de manière non limitative, un saut à l'étape (ETP002) de choix du type de paramétrage. L'utilisateur peut aussi choisir de quitter le paramétrage (cond0102) pour activer le module d'analyse ou quitter les menus concernant les alertes et utiliser le dispositif portable pour d'autres usages.

De manière non limitative, les étapes d'initialisation ou de réglage d'un module d'alerte, d'un module de surveillance ou d'un module d'évaluation, sont exécutées par l'utilisateur, via l'interface (IHM) homme machine ou de façon automatique, par le module (106) d'apprentissage ou à distance par le module (105) de commande à distance. Le module (106) d'apprentissage réalise, par exemple, les choix de paramétrage en fonction de son programme d'examen des séquences d'évènement informatiques ou en fonction de commandes reçues par le module (105) de commande à distance et provenant d'un système expert, d'un réseau local ou du réseau Internet.

L'ordre des différentes étapes relatives aux alertes, décrites précédemment, n'est pas limitatif. Une de ces étapes pourra aussi être exécutée seule. Une étape de sauvegarde en bibliothèque pourra, par exemple, être exécutée seule pour enrichir le dispositif d'alerte en situation de mobilité et éventuellement le système central expert.

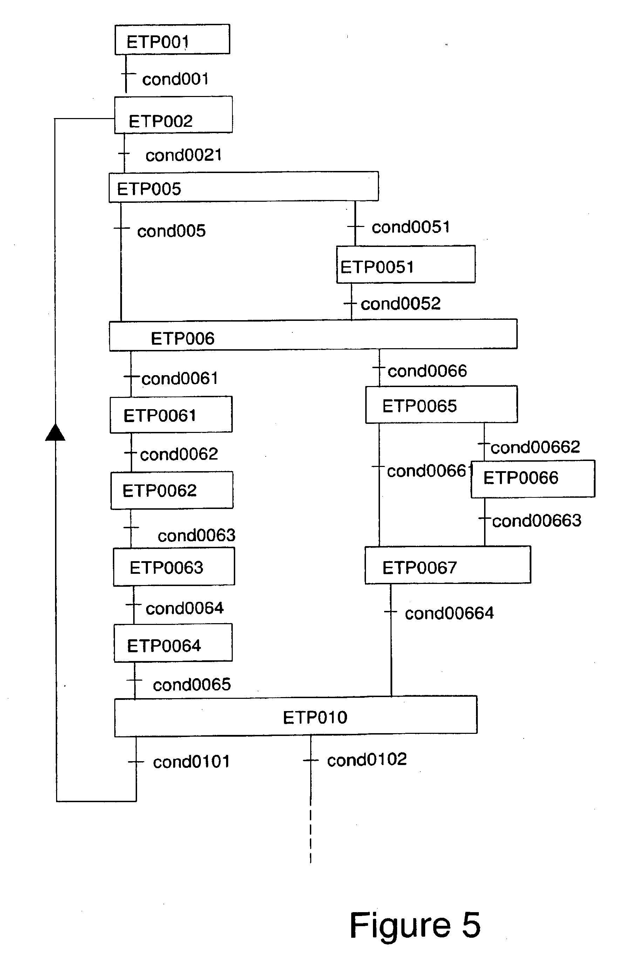

Un exemple de paramétrage d'un module de surveillance va maintenant être décrit. La figure 5 représente un exemple de procédé de configuration d'un déclencheur. A l'étape (ETP002) de choix du type de paramétrage, l'utilisateur choisit, par exemple, via l'interface (IHM) homme machine, le paramétrage des déclencheurs (cond0021).

De manière non limitative, le module (104) de paramétrage propose (ETP005), par exemple, un menu de sélection transmis par l'interface (IHM) utilisateur, pour choisir (cond005) un des déclencheurs associés au module (101) d'analyse ou pour choisir (cond0051) la création d'un nouveau module (M01) de surveillance associé au module (101) d'analyse.

Après le choix (cond005) d'un module (M01) de surveillance existant ou le choix (cond0051) de la création d'un nouveau module de surveillance et son enregistrement (cond0052), le module (104) de paramétrage exécute, par exemple, une étape (ETP006) de configuration du module sélectionné. De manière non limitative, l'étape de création (ETP0051) d'un nouveau module de surveillance est réalisée à partir d'une bibliothèque mémorisée de modules de surveillance ou un module de surveillance vierge ou par défaut est créé. De manière non limitative, des données représentatives du contexte sont utilisées pour l'initialisation du nouveau module de surveillance.

A l'étape (ETP006) de configuration du module (M01) de surveillance sélectionné, l'utilisateur, choisit, par exemple, via l'interface (IHM) homme machine, le paramétrage (cond0061) du module de surveillance. De manière non limitative, le module (104) de paramétrage exécute alors une étape (ETP0061) de choix du capteur (CM01). Le capteur est, par exemple, choisi dans un menu de sélection affiché par le module (104) de paramétrage, via l'interface utilisateur. L'utilisateur peut, par exemple, choisir l'utilisation d'un bouton ou d'une combinaison de bouton comme déclencheur. L'utilisateur paramètre ainsi son dispositif portable de communication pour pouvoir déclencher une alerte très rapidement par un appui sur un ou plusieurs boutons de son choix.

Selon un exemple non limitatif, l'utilisateur choisit le micro (MIC) comme capteur déclencheur. Les sons captés par le micro sont alors traités, lorsque le module (101) d'analyse est activé, pour détecter un mot ou une phrase de déclenchement. Ainsi l'utilisateur peut paramétrer son dispositif portable de communication pour que le fait de prononcer le mot « danger », lorsque le module (101) d'analyse est activé, soit un déclencheur d'une alerte déterminée. De façon avantageuse un utilisateur effectuant un travail manuel comportant des risques pourra déclencher son dispositif de communication à distance sans avoir à manipuler le clavier ou appuyer sur un bouton. De manière non limitative, la phrase ou le mot déclencheur sont quelconques et peuvent être enregistrés par l'utilisateur.

Selon un exemple non limitatif, l'utilisateur choisit un lecteur radiofréquence, par exemple, du type RFID (Radio Frequency IDentification), comme capteur déclencheur. Le lecteur RFID est par exemple intégré au dispositif portable de communication et associé à une carte RFID transportée par l'utilisateur avec son dispositif portable de communication. Le lecteur radiofréquence émet par exemple un signal comprenant une énergie d'alimentation de la carte radiofréquence et des données de commande pour que la carte radiofréquence transmette, en réponse, son numéro d'identification. Le lecteur radiofréquence peut ainsi détecter l'absence ou la présence de la carte radiofréquence de l'utilisateur. Dans le cas où la carte radiofréquence transmet son numéro d'identification, en réponse à la requête du lecteur, le lecteur produit une variable représentative de la présence de cette carte. Dans le cas, par exemple, où le téléphone est volé ou perdu, le lecteur détecte l'absence de !a carte de l'utilisateur, permettant ainsi de déclencher un module d'alerte antivol incapacitant, par exemple, les fonctions du dispositif portable de communication ou demandant l'entrée d'un code secret, par le clavier, pour sortir de l'état d'alerte et autoriser de nouveau le fonctionnement non restreint du dispositif portable.

Selon un exemple non limitatif, l'utilisateur choisit un capteur biométrique comme capteur déclencheur, le capteur biométrique étant connecté par le connecteur au dispositif portable de communication. De manière non limitative, un ou plusieurs capteurs biométriques sont reliés au dispositif (1) portable de communication qui peut alors servir pour réaliser un diagnostic précoce ou une télésurveillance de l'utilisateur couplé au dispositif d'alerte.

Selon un exemple non limitatif, l'ensemble des données représentatives des paramètres mesurés par les capteurs est transmis de façon périodique à un serveur de surveillance, le dispositif portable de communication étant, par exemple, équivalent à une centrale de mesures ambulatoire. Les capteurs sont par exemple, des capteurs biométriques portés par l'utilisateur, de type casque, bandeau, gilet, ceinture, bracelet ou chaussure. Ces différents capteurs fournissent par exemple des données représentatives de caractéristiques biométriques pour réaliser des tests de vigilance ou une analyse comportementale.

Selon un exemple non limitatif, le capteur choisi par l'utilisateur est un capteur produisant des données représentatives de l'environnement de l'utilisateur. Ce capteur est, par exemple, un capteur réalisant une analyse chimique pour déterminer la présence d'un gaz toxique, le taux d'oxygène ou encore un capteur mesurant la température de l'air ou la radioactivité. Ainsi un pompier est non seulement averti en cas de présence d'un gaz toxique, mais un message est aussi envoyé à un poste de surveillance ou à un coéquipier, pour que le pompier soit assisté le plus rapidement possible. De plus, en cas d'alerte la combinaison des capteurs environnementaux et des capteurs biométriques permet de transmettre des paramètres pour surveiller le pompier et prévoir les risques. Une communication est, par exemple, établie en cas de danger, les informations nécessaires à l'évaluation du danger étant transmises avant ou en même temps que l'établissement de la communication.

Le module (104) de paramétrage enregistre, par exemple, dans le module (M01) de surveillance, un pointeur vers une interface ou un espace mémoire correspondant au capteur choisi. Après l'enregistrement (cond0062) du capteur associé au module de surveillance, le module (104) de surveillance réalise une étape (ETP0062) de choix du traitement préliminaire des données produites par le capteur. Une fonction de traitement est par exemple choisie dans une bibliothèque de fonctions de traitement, stockée en mémoire. De manière non limitative, la fonction de traitement est, par exemple, une fonction de recopie de la donnée produite par le capteur, la variable (V01) d'alerte associée au module de surveillance étant réglée avec cette donnée.

Selon un autre exemple, la fonction de traitement préliminaire réalise une opération paramétrable de mise en forme ou de mise à l'échelle de la donnée reçue avant la transmission à l'unité de calcul pour le positionnement de la variable d'alerte. La mise en forme comprend par exemple, la suppression d'un en-tête ou d'une partie d'identification du capteur et la mise à l'échelle comprend, par exemple, la multiplication par un facteur ou la soustraction d'une valeur de référence. De manière non limitative, la fonction de traitement préliminaire réalise une ou plusieurs pondérations paramétrables.

Selon un autre exemple, la fonction de traitement préliminaire réalise une intégration ou une dérivation du paramètre reçu pour transmettre le résultat à l'unité de calcul afin de régler la variable d'alerte associée au module de surveillance. De manière non limitative, la fonction de traitement peut aussi réaliser un traitement du signal, par exemple, par filtre paramétrable, appliqué à la donnée numérique produite par le capteur.

Selon un autre exemple, la fonction de traitement préliminaire de la donnée produite par le capteur, est une fonction de comparaison avec une valeur de référence paramétrable pour adapter le seuil du déclenchement d'alerte et régler la variable d'alerte dans un état représentatif d'une urgence ou représentatif d'un état normal.

Après l'enregistrement de la fonction de traitement préliminaire (cond0063), le module (104) de paramétrage exécute, par exemple, une étape (ETP0063) d'initialisation des paramètres de la fonction de traitement préliminaire. L'utilisateur peut, par exemple, choisir, par un menu de sélection, les paramètres de la fonction de traitement.

Ces paramètres sont, par exemple, enregistrés (cond0064) par le module (104) de paramétrage dans le module (M01) de surveillance, puis le module (104) de paramétrage exécute, de manière non limitative, une étape (ETP0064) de définition d'une temporisation. La temporisation peut être, par exemple, réglée pour retarder l'effet d'une donnée de déclenchement ou la temporisation peut être réglée nulle pour supprimer le retard de déclenchement. De manière non limitative, une donnée de déclenchement peut ne pas être prise en compte si le facteur déclenchant se produit sur une durée inférieure à la durée de temporisation.

Selon un autre exemple, la temporisation peut être utilisée seule sans capteur déclencheur, la variable d'alerte étant représentative d'une temporisation en cours ou de la fin d'une temporisation, la fin de temporisation déclenchant une alerte.

Le module (104) de paramétrage exécute, par exemple, après l'enregistrement (cond0065) de la durée de temporisation, une étape (ETP010) de choix de sortie ou non du paramétrage.

La suppression d'un module de surveillance va maintenant être décrite. De manière non limitative, à l'étape (ETP006) de configuration du module sélectionné, si le choix est de supprimer ce module (cond0066), une étape (ETP0065) suivante est exécutée dans laquelle le module (104) de paramétrage propose un enregistrement avant la suppression du module sélectionné. L'utilisateur peut alors choisir de supprimer directement le module de surveillance (cond00661) ou de réaliser (cond00662) une sauvegarde auparavant. Une étape (ETP0066) de sauvegarde est, par exemple, réalisée pour intégrer le module de surveillance sélectionné, dans une bibliothèque de modules de surveillance, stockée en mémoire (DATA). La bibliothèque de modules de surveillance peut ainsi être enrichie par des modules configurés au cas par cas. Le module de surveillance nouvellement stocké est par exemple, associé à un nom de surveillance, entré par l'utilisateur, ou à un contexte du dispositif portable de communication, utilisés, par exemple à l'initialisation.

Après l'enregistrement (cond00663) dans la bibliothèque, la suppression (ETP0067) est commandée, par exemple, par le module (104) de paramétrage. A la fin de la suppression (cond00664) le module (104) de paramétrage exécute par exemple l'étape (ETP010) proposant à l'utilisateur de continuer ou de quitter le paramétrage.

L'ordre des différentes étapes relatives à la surveillance, décrites précédemment, n'est pas limitatif. Une de ces étapes pourra aussi être exécutée seule. Une étape de sauvegarde en bibliothèque pourra, par exemple, être exécutée seule pour enrichir le dispositif d'alerte en situation de mobilité et éventuellement le système central expert.

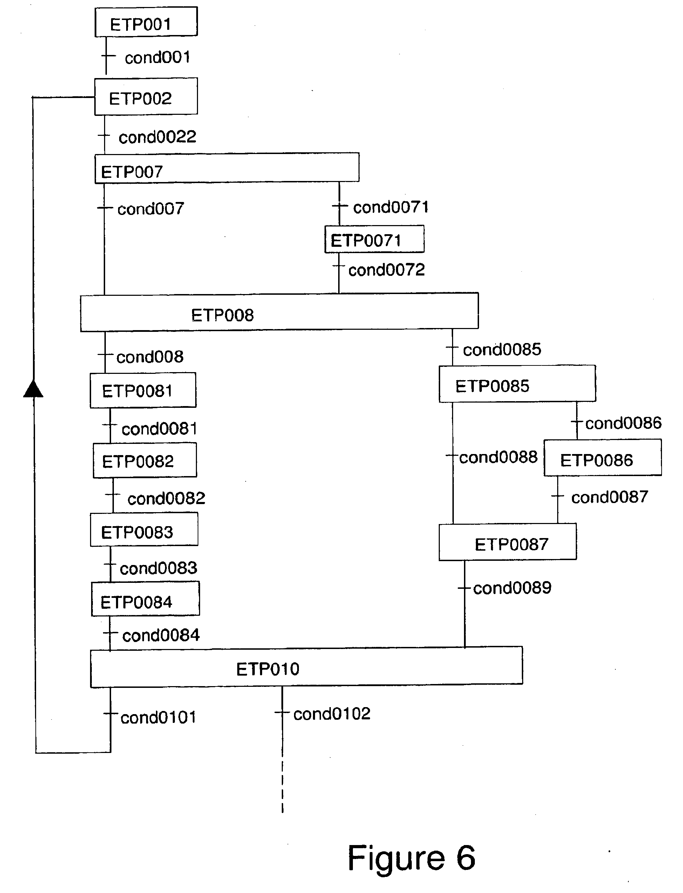

Un exemple de paramétrage d'un module d'évaluation va maintenant être décrit. Comme représenté à la figure 6, à l'étape (ETP002) de choix du type de paramétrage, l'utilisateur peut choisir, via l'interface homme machine (IHM), le paramétrage des scénarii (cond0022). De manière non limitative, le module (104) de paramétrage exécute, ensuite une étape (ETP007) de sélection d'un scénario. Le module (104) de paramétrage propose par exemple, de sélectionner un des scénarii (cond007) existants ou de créer un nouveau scénario (cond0071), par un menu de sélection.

De manière non limitative, le module (104) de paramétrage exécute une étape (ETP0071) de création d'un nouveau module (C102) d'évaluation choisi, par exemple, dans une bibliothèque mémorisée de modules d'évaluation. Le module d'évaluation est, par exemple, un module standard ou vierge ou par défaut. De manière non limitative, des données représentatives du contexte sont utilisées pour l'initialisation du nouveau module d'évaluation.

Après la création (cond0072) du nouveau module d'évaluation, l'étape (ETP008) de configuration du scénario est par exemple exécutée par le module (104) de paramétrage.

L'étape (ETP008) de configuration du scénario sélectionné, donne, par exemple, le choix à l'utilisateur entre la modification (cond008) du scénario sélectionné ou sa suppression (cond0085). Le choix (cond0085) de la suppression implique par exemple, que le module (C102) d'évaluation correspondant à ce scénario sera supprimé par le module (104) de paramétrage. De manière non limitative, une étape (ETP0085) proposant de sauvegarder le scénario est exécutée, par le module (104) de paramétrage. Le module de paramétrage peut ainsi enregistrer (ETP0086) le module d'évaluation dans une bibliothèque si l'utilisateur entre le choix (cond0086) de sauvegarder le scénario. Cette sauvegarde permet, par exemple, à l'utilisateur de supprimer un scénario de l'ensemble des scénarii exécutés simultanément dans le module (101) d'analyse sélectionné, tout en conservant ce modèle de scénario, par exemple pour d'autres modules (101) d'analyse. La bibliothèque de modules d'évaluation peut ainsi être enrichie par des modules configurés au cas par cas. Le module d'évaluation nouvellement stocké est par exemple, associé à un nom d'évaluation entré par l'utilisateur, ou à un contexte du dispositif portable de communication, utilisé, par exemple à l'initialisation. Après la sauvegarde (cond0087) la suppression (ETP0087) est réalisée.

Si l'utilisateur choisit de ne pas sauvegarder (cond0088) le module d'évaluation sélectionné, l'étape de suppression (ETP0087) est directement exécutée par le module (104) de paramétrage. De manière non limitative, une étape (ETP010) de choix de sortie ou non du paramétrage est réalisée à la fin de la suppression (cond0089) du module d'évaluation.

Si à l'étape (ETP008) de configuration du scénario l'utilisateur choisit, par le menu de sélection, le paramétrage (cond008) du scénario, le module (104) de paramétrage, interprète la commande utilisateur et réalise, de manière non limitative, une étape (ETP0081) de choix d'une fonction d'analyse. Le module (C102) d'évaluation sélectionné est, par exemple, modifié par un réglage de sa fonction d'analyse. L'utilisateur choisit, par exemple, dans une bibliothèque de fonctions d'analyse, la fonction ou le programme utilisé pour évaluer le niveau d'alerte.

De manière non limitative, un programme d'évaluation comprend plusieurs étapes d'attente conditionnelle en fonction de tests comparatifs, selon différents seuils paramétrables, pour plusieurs variables d'alerte, le dernier test du programme d'évaluation, réalisant le déclenchement d'un module d'alerte.

Selon un autre exemple, chaque étape d'attente conditionnelle est suivie de l'activation d'un module (102) d'alerte distinct pour réaliser des envois d'alertes selon des niveaux d'urgence croissants. Un premier test de dépassement d'une température est par exemple réalisé pour envoyer, en cas de dépassement, une alerte de niveau bas. Puis un deuxième test de dépassement d'une deuxième température plus élevée, peut être réalisé pour envoyer une alerte de niveau moyen. Enfin un dernier test de présence de gaz toxique est réalisé, pour déclencher une alerte de niveau élevé. Plusieurs programmes d'évaluation différents sont, par exemple, fournis avec des paramètres configurables initialisés par défaut.

Selon un autre exemple, la fonction d'évaluation est une fonction réalisant une opération conditionnelle sur plusieurs variables, chaque variable étant pondérée par un coefficient paramétrable. Le résultat de la fonction d'évaluation déclenche, par exemple, l'activation d'un module d'alerte déterminé, par le module d'évaluation.

Selon un autre exemple, la fonction d'évaluation réalise un test d'appartenance à un ensemble paramétrable correspondant à plusieurs cas de déclenchement d'alerte, sur une ou plusieurs variables d'alerte. Les résultats des fonctions de traitement préliminaire sont ainsi évalués selon leur appartenance à cet ensemble représentatif de situations dangereuses.

Après avoir initialisé (cond0081) la fonction ou le programme d'évaluation, le module (104) de paramétrage réalise, de manière non limitative, une étape (ETP0082) de paramétrage de la fonction ou du programme du module (C102) d'évaluation. Un menu de sélection propose par exemple, à l'utilisateur de rentrer les différents paramètres de la fonction d'évaluation ou de choisir les paramètres dans une liste.

Après l'enregistrement dans le module (C102) d'évaluation, des paramètres de configuration (cond0082), le module (104) de paramétrage exécute, de manière non limitative, une étape (ETP0083) d'association d'un ou plusieurs modules (102) d'alerte avec le module (C102) d'évaluation. Cette étape permet de choisir quelle alerte sera envoyée selon le scénario sélectionné. Un pointeur du module (C102) d'évaluation est, par exemple, réglé pour pointer sur un module (102) d'alerte sélectionné.

Après l'enregistrement (cond0083) de la ou des alertes associées, le module (104) de paramétrage exécute, de manière non limitative, une étape (ETP0084) d'initialisation des paramètres d'entrée de la fonction d'évaluation. La fonction ou le programme d'évaluation réalise en effet un calcul en fonction des valeurs d'une ou plusieurs des variables d'alerte (V01, V02, V03). La définition des entrées permet, par exemple, à l'utilisateur d'utiliser n'importe quelle variable produite par un capteur. La donnée représentative d'un paramètre à surveiller, est par exemple mise en forme par un module de surveillance et pondérée par la fonction d'évaluation, par exemple, si le capteur n'est pas un capteur standard.

De plus les paramètres d'une fonction ou d'un programme d'évaluation peuvent être modifiés de façon automatique pour réaliser un paramétrage plus précis, suite par exemple, à un déclenchement excessif détecté (cond0132).

Après l'enregistrement (cond0084) par exemple d'un tableau de pointeurs vers les variables d'alertes testées, le module (104) de paramétrage exécute par exemple, l'étape (ETP010) proposant de sortir du mode de paramétrage.

L'ordre des différentes étapes relatives aux scénarii, décrites précédemment, n'est pas limitatif. Une de ces étapes pourra aussi être exécutée seule. Une étape de sauvegarde en bibliothèque pourra, par exemple, être exécutée seule pour enrichir le dispositif d'alerte en situation de mobilité et éventuellement le système central expert.

Un module (106) d'apprentissage dont la fonction d'adaptation automatique est activée, pourra par exemple modifier un module d'évaluation pour prendre en compte une entrée supplémentaire. L'examen, par le module d'apprentissage, des séquences d'évènements informatiques comprenant des données représentatives de sons enregistrés, révèle par exemple, qu'un cri avec un volume sonore supérieur à un seuil déterminé, a été poussé. Cet évènement non prévu initialement dans le ou les scénarii du module d'analyse, est par exemple, introduit par une modification d'un module d'évaluation. Le module d'évaluation modifié a par exemple, une entrée supplémentaire, ou en remplacement d'une précédente, correspondant aux sons perçus. Les sons perçus sont, par exemple, comparés à un ensemble de sons reconnaissables ou sont traités selon leur volume sonore.

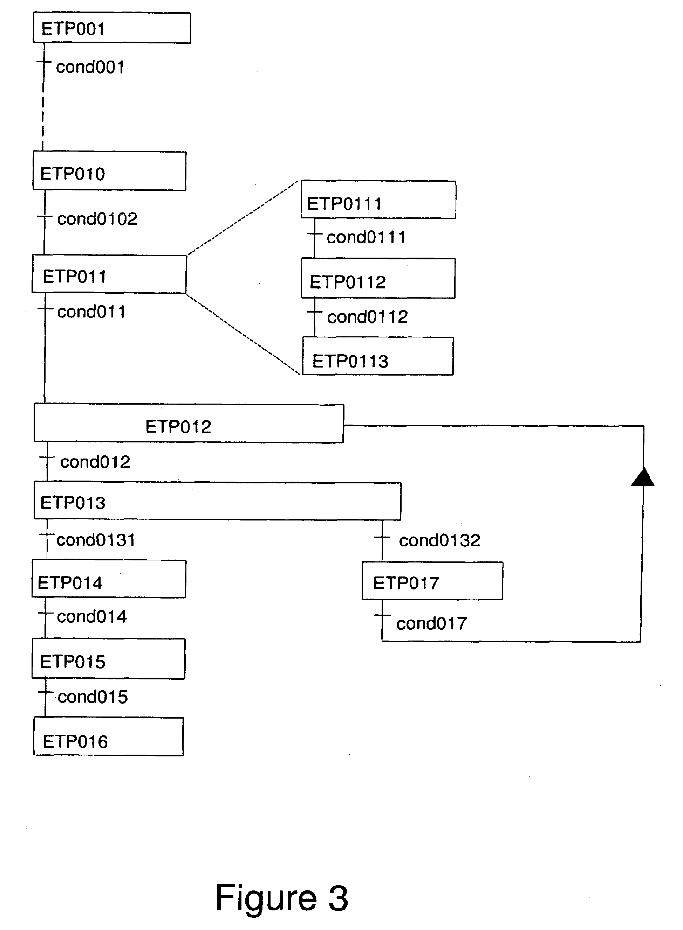

Un exemple d'activation d'un module d'analyse, correspondant à un contexte d'utilisation, va maintenant être décrit. En sortie du paramétrage (cond0102) un module d'analyse (101) est, par exemple, activé (ETP011). La figure 3 donne un exemple non limitatif de procédé d'alerte. De manière non limitative, l'activation du module (101) d'analyse, permettant d'évaluer un niveau d'urgence, est réalisée par l'utilisateur via l'interface (IHM) homme machine ou par le module (105) d'activation à distance recevant un signal de commande d'activation ou par le module (106) d'apprentissage examinant les séquences d'évènements informatiques.

Selon un exemple non limitatif de réalisation, l'activation du module (101) d'analyse comprend une étape (ETP0111) de paramétrage préliminaire, permettant de régler des paramètres de modules d'évaluation, de surveillance ou d'alerte. Ce paramétrage préliminaire réalisé juste avant l'activation du module d'analyse, permet notamment de régler un ou plusieurs paramètres précisant le contexte d'utilisation. Après l'entrée (cond0111) de ces paramètres, les paramètres des modules concernés sont enregistrés à l'étape suivante (ETP0112). Après l'enregistrement (cond0112) des paramètres, l'activation du module d'analyse est commandée à l'étape suivante (ETP0113). De cette façon, par exemple, un enfant surveillé lors d'un trajet, pourra entrer le nom de la personne à prévenir en cas de problème. L'enfant pourra, par exemple, choisir ce jour là :
- de prévenir son père par un courrier électronique, sur son ordinateur de bureau, ou
- de prévenir sa mère par un message texte, de type SMS, sur son téléphone cellulaire, ou
- de paramétrer la durée du trajet en fonction d'une destination proposée via l'interface (IHM) utilisateur.

Après que le module (101) d'analyse soit passé de son état dormant à son état actif (cond011), une évaluation (ETP012) du niveau d'urgence est réalisée. Le ou les scénarii du module (101) d'analyse sont, en effet, appliqués pour détecter si une condition de déclenchement d'une alerte est présente. De manière non limitative, le module (106) d'apprentissage réalise l'enregistrement de séquences d'évènements informatiques correspondant, par exemple, aux états des variables d'alerte en concordance de phase avec les états des modules de surveillance, d'évaluation et d'alerte. Cet enregistrement, par exemple, dans un espace mémoire associé au module (106) d'apprentissage, peut être transmis par la suite, par les moyens de communication, pour être analysé ou peut être examiné pour déclencher des commandes de configuration des modules de surveillance, d'évaluation ou d'alerte.

De manière non limitative, le module (106) d'apprentissage exécute, en parallèle du module d'analyse et de ses modules logiciels associés, un programme d'examen des séquences mémorisées pour agir, par la suite, sur les modules logiciels associés au module d'analyse.

De manière non limitative, l'utilisateur peut bloquer l'autoévaluation, lorsque le module d'analyse s'est révélé suffisamment fiable. Le blocage permet de réaliser une analyse déterminée connue à l'avance. L'utilisateur bloque, par exemple, le module d'apprentissage, via l'interface (IHM) homme machine.

La détection d'un état d'urgence (cond012) provoque, par exemple, une étape (ETP013) d'activation d'un module d'alerte, par exemple pour l'émission d'un message d'avertissement. De manière non limitative, ce message d'avertissement est suivi d'une annulation (cond0132) de l'état d'urgence, réalisée par exemple, par l'utilisateur, par l'appui sur une combinaison déterminée de touches. L'annulation est, par exemple, suivie par une étape (ETP017) de désactivation de l'analyse et de modification de paramètres, par exemple d'un programme d'évaluation ou d'un module de surveillance associé au module d'analyse. Un paramètre du module d'évaluation ou de surveillance, comme par exemple un seuil de dépassement, ayant déclenché le message d'avertissement, est par exemple modifié pour augmenter la plage de tolérance avant déclenchement d'une alerte.

Après ces modifications (cond017) du ou des modules logiciels, l'analyse est réactivée, le module (101) d'analyse réalisant de nouveau une évaluation (ETP012) du niveau d'urgence, correspondant à un contexte précisé.

Dans un autre cas, si une confirmation (cond0131) d'urgence est détectée après l'envoi (ETP013) du message d'avertissement, une étape (ETP014) d'envoi d'un message d'urgence est, par exemple, réalisée. La confirmation correspond, par exemple, à l'écoulement d'une temporisation sans détection d'action d'annulation par l'utilisateur.

L'étape d'envoi (ETP014) d'un message d'urgence correspond par exemple à l'activation d'un module d'alerte (102) par un module (C102) d'évaluation. De manière non limitative, après l'envoi (cond014) du message d'alerte, le module d'alerte activé bloque (ETP015) le dispositif de communication dans un état d'urgence. Un message est, par exemple, affiché à l'écran pour inviter l'utilisateur à entrer un code secret, tandis que les fonctions, par exemple liées à la téléphonie, sont interdites dans cet état d'urgence.

Une sortie de l'état d'urgence (cond015) est par exemple réalisée lorsque l'utilisateur entre son code secret au clavier ou réalise une autre procédure de sortie d'état d'urgence. De manière non limitative, la détection (cond015) de la sortie d'état d'urgence, est suivie par la désactivation (ETP016) du module (101) d'analyse.

De façon avantageuse, un module d'analyse avec ses modules associés, pourra être stocké dans une bibliothèque de modules d'analyse. La bibliothèque de modules d'analyse comprend des modules d'analyse standard ou adaptés à un usage déterminé pour être enrichie par des modules paramétrés au cas par cas. Le module d'analyse stocké est, par exemple, associé à un nom entré par l'utilisateur ou à des données représentatives d'un contexte d'utilisation du dispositif portable, utilisées lors de l'initialisation. L'utilisateur pourra ainsi initialiser le dispositif portable dans une configuration opérationnelle pour réaliser une surveillance afin de déclencher une procédure d'urgence.

De façon avantageuse, la fonction d'adaptation automatique du module (106) d'apprentissage, permet au dispositif portable d'alerte de prendre en compte des évènements non préprogrammés. Le module (106) d'apprentissage enregistre, en effet des séquences d'évènements informatiques qui constituent une base de connaissance capitalisant de l'expérience. De cette façon le dispositif portable d'alerte n'est pas limité, dans ses programmes d'évaluation, à un arbre figé d'évènements successifs, mais l'arbre des évènements peut évoluer en prenant en considération une nouvelle entrée surveillée. Des programmes d'examen des séquences d'évènements informatiques pourront être fournis par un fabricant, pour un usage déterminé, puis être mis à jour, pour profiter de l'expérience acquise par le dispositif portable d'alerte. Les mises à jour peuvent aussi provenir d'un système central expert capitalisant les expériences de plusieurs dispositifs portables d'alerte.

Il doit être évident pour les personnes versées dans l'art que la présente invention permet des modes de réalisation sous de nombreuses autres formes spécifiques sans l'éloigner du domaine d'application de l'invention comme revendiqué. Par conséquent, les présents modes de réalisation doivent être considérés à titre d'illustration, mais peuvent être modifiés dans le domaine défini par la portée des revendications jointes, et l'invention ne doit pas être limitée aux détails donnés ci-dessus.

## Revendications

1. Procédé d'alerte exécuté par un dispositif portable en situation de mobilité associé à au moins un réseau de télécommunication, le dispositif portable comprenant au moins des moyens (UC) de traitement, des moyens (MEM) de mémorisation et des moyens (A, DM, M) de réception ou d'émission de données par le réseau, le procédé comprenant au moins :
- une étape (ETP0081) d'initialisation d'au moins un module (101) d'analyse, initialisé en fonction de données représentatives d'un contexte d'utilisation déterminé comprenant au moins des données représentatives d'au moins un état d'au moins un capteur, le module d'analyse comprenant au moins un module d'évaluation évaluant au moins une variable d'alerte (V01, V02, V03),
- une étape (ETP084) d'initialisation d'au moins un module (M01, M02, M03) de surveillance initialisé en fonction du contexte d'utilisation, le module de surveillance réglant la variable d'alerte évaluée dans un état représentatif de l'état dudit capteur ou d'un autre capteur (CM01, CM02), associé au module de surveillance,
- une étape (ETP0083) d'initialisation d'au moins un module (102, 1021, 1022) d'émission d'alerte initialisé en fonction du contexte d'utilisation, commandé en fonction de l'évaluation de la variable d'alerte par le module d'évaluation et déclenchant, lors de son activation, au moins l'envoi d'un message d'alerte par les moyens d'émission de données par le réseau ou par d'autres moyens (T102) d'émission,
- une étape de réglage préliminaire à l'activation, d'au moins le module d'évaluation, de surveillance ou d'émission d'alerte, le réglage étant réalisé par l'interface utilisateur,
- une étape (ETP011) d'activation du module d'analyse et de son module d'évaluation,
- une étape de réglage de la variable d'alerte par le module de surveillance associé au contexte,
- une étape (ET014) d'activation du module d'émission d'alerte lorsque le module d'évaluation détecte un état d'urgence.

2. Procédé d'alerte selon la revendication 1, **caractérisé en ce que** l'étape d'initialisation du module (101) d'analyse, du module de surveillance ou du module d'alerte réalisée en fonction d'au moins une donnée représentative du contexte d'utilisation, comprend la comparaison d'au moins cette donnée avec des données d'initialisation associées à des modules d'une bibliothèque.

3. Procédé d'alerte selon la revendication 2, **caractérisé en ce qu'**il comprend une étape d'archivage du module d'analyse, du module de surveillance ou respectivement du module d'émission d'alerte, dans la bibliothèque de modules, comprenant le stockage de données représentatives du module (101) d'analyse, du module de surveillance ou respectivement du module (102) d'émission d'alerte, associées à des données représentatives d'un nom entré par l'utilisateur ou de données représentatives du contexte d'utilisation.

4. Procédé d'alerte selon l'une des revendications 1 à 3, **caractérisé en ce que** l'activation du module d'analyse est réalisée concomitante au stockage, par un module (106) d'apprentissage, de séquences d'évènements informatiques représentatives des états du module d'analyse, de surveillance et d'émission d'alerte, en concordance de phase avec les états des variables d'alerte.

5. Procédé d'alerte selon l'une des revendications 1 à 4, **caractérisé en ce que** l'étape (ETP011) d'activation du module d'analyse et de son module d'évaluation est suivie d'au moins une étape de réglage du module (101) d'analyse, du module de surveillance ou du module (102) d'émission d'alerte, en fonction de :
- une commande de paramétrage manuel entrée par une interface (IHM) utilisateur, ou
- une commande de paramétrage automatique produite par le module d'apprentissage en fonction d'une annulation de l'état d'urgence entrée par l'interface utilisateur.

6. Procédé d'alerte selon la revendication 5, **caractérisé en ce que** le réglage du module d'analyse comprend le réglage de son module d'évaluation par :
- un choix d'un programme d'évaluation dans une bibliothèque de programmes, ou
- un paramétrage du programme d'évaluation choisi, ou
- une association d'un ou plusieurs modules d'émission d'alerte commandés en fonction de l'évaluation, ou
- un réglage d'une ou plusieurs références de variables d'alerte lues en entrée du programme d'évaluation.

7. Procédé d'alerte selon la revendication 5 ou 6, **caractérisé en ce que** le réglage du module de surveillance comprend :
- un réglage d'une référence d'un capteur surveillé, ou
- un choix d'une fonction de traitement préliminaire réglant la variable d'alerte, ou
- un paramétrage de la fonction de traitement préliminaire, ou
- un réglage d'une temporisation de déclenchement d'alerte.

8. Procédé d'alerte selon l'une des revendications 5 à 7, **caractérisé en ce que** le réglage du module d'émission d'alerte comprend :
- un choix du type de message, ou
- un réglage du contenu du message, ou
- un réglage d'au moins une référence de variable (V01, V02, V03) d'alerte ou de séquence d'évènements informatiques insérée au contenu du message d'alerte envoyé, lors de l'activation du module d'émission d'alerte, ou
- un réglage de la destination du message, ou
- un réglage de paramètres de déblocage et de sortie d'un état d'alerte.

9. Procédé d'alerte selon la revendication 4, **caractérisé en ce que** l'étape d'activation du module (102) d'émission d'alerte est suivie d'au moins :
- une étape de désactivation du module (C102) d'évaluation,
- une étape de réglage automatique, par le module (106) d'apprentissage, du module (101) d'évaluation ou de son module de surveillance associé ou de son module (102) d'émission d'alerte associé
- une étape d'activation du module (C102) d'évaluation.

10. Procédé d'alerte selon la revendication 9, **caractérisé en ce que** l'étape de désactivation du module (C102) d'évaluation est précédée d'au moins :
- une étape d'envoi d'un message d'avertissement, vers l'interface (IHM) utilisateur,
- une étape d'attente d'une entrée d'annulation de l'état d'urgence, par l'interface utilisateur,
- une étape de détection de l'entrée d'annulation suivie de l'étape de désactivation du module (C102) d'évaluation.

11. Procédé d'alerte selon l'une des revendications 1 à 10, **caractérisé en ce que** le module (C102) d'évaluation exécute au moins :
- une étape de temporisation avant validation de l'état d'urgence, débutant au dépassement, par la variable testée, d'un seuil paramétrable et s'interrompant dans le cas d'un retour de la variable testée sous le seuil.

12. Procédé d'alerte selon l'une des revendications 1 à 11, **caractérisé en ce que** l'activation du module d'émission d'alerte déclenche un appel téléphonique à l'aide d'un microphone, d'un haut-parleur ou d'une caméra.

13. Dispositif portable en situation de mobilité associé à un réseau de télécommunication, comprenant au moins :
- des moyens (A, DM, M) de réception ou d'émission de données par le réseau,
- des moyens (UC) de traitement,
- des moyens (MEM) de mémorisation
mémorisant un contexte d'utilisation comprenant des données représentatives d'au moins un état d'au moins un capteur, les moyens de traitement associés aux moyens de mémorisation sont agencés de façon à réaliser au moins :
un module (101) d'analyse, initialisé en fonction du contexte d'utilisation et commandé dans un état actif ou dormant par un module (103) de commande,
le module d'analyse comprenant au moins un module (C102) d'évaluation commandant au moins un module d'émission d'alerte initialisé en fonction du contexte, en fonction d'au moins une variable d'alerte réglée dans un état représentatif d'un capteur, par un module (M01, M02, M03) de surveillance initialisé en fonction du contexte,
au moins un module parmi le module d'analyse, le module d'émission d'alerte et le module de surveillance, étant réglé par l'utilisateur préliminairement à son activation,
le module (101) d'analyse, dans son état actif, commandant par son module (C102) d'évaluation, l'activation du module d'émission d'alerte si la ou les variables testées sont représentatives d'un état d'urgence, le module (102) d'émission d'alerte activé déclenchant l'envoi d'au moins un message d'alerte, par les moyens d'émission de données par le réseau ou par d'autres moyens d'émission.

14. Dispositif portable selon la revendication 13, **caractérisé en ce qu'**il comprend un module (104) de paramétrage réglant le module d'évaluation, de surveillance ou d'émission d'alerte, lorsque le module d'analyse est dans son état dormant, le module de paramétrage étant piloté par :
- une interface (IHM) utilisateur, ou
- un module (106) d'apprentissage, ou
- un module (105) de commande à distance.

15. Dispositif portable selon la revendication 14, **caractérisé en ce que** le module (106) d'apprentissage dans son état actif mémorise des séquences d'évènements informatiques représentatives des états des modules de surveillance, d'évaluation et d'émission d'alerte, en concordance de phase avec les états des variables (V01, V02, V03) d'alerte.

16. Dispositif portable selon la revendication 15, **caractérisé en ce qu'**un signal de commande à distance est reçu par le module (105) de commande à distance qui pilote le module (101) d'analyse à l'état actif ou dormant.

17. Dispositif portable selon l'une des revendications 13 à 16, **caractérisé en ce qu'**il comprend une bibliothèque de modules d'analyse, d'évaluation, de surveillance ou d'émission d'alerte dédiés chacun à une activité déterminée ou de modules d'analyse, d'évaluation, de surveillance ou d'émission d'alerte sauvegardés chacun avec des données associées d'initialisation représentatives du contexte d'utilisation lors de la sauvegarde.

18. Dispositif portable selon l'une des revendications 13 à 17, **caractérisé en ce qu'**il comprend au moins un capteur environnemental communiquant, à son module de surveillance associé, un signal représentatif d'une caractéristique physique ou chimique d'un environnement dans lequel se trouve le dispositif portable.

19. Dispositif portable selon l'une des revendications 13 à 18, **caractérisé en ce qu'**il comprend au moins un capteur biométrique communiquant, à son module de surveillance associé, un signal représentatif d'une caractéristique physiologique de l'utilisateur du dispositif portable.

20. Dispositif portable selon l'une des revendications 13 à 19, **caractérisé en ce qu'**il comprend au moins un capteur (LOC) localisateur communiquant, à son module de surveillance associé, un signal représentatif d'une localisation géographique à laquelle se trouve le dispositif portable.

21. Dispositif portable selon l'une des revendications 13 à 20, **caractérisé en ce qu'**il comprend au moins un capteur de présence d'une carte personnelle communiquant, à son module de surveillance associé, un signal représentatif de la présence de la carte personnelle à proximité du dispositif portable.

22. Dispositif portable selon l'une des revendications 13 à 21, **caractérisé en ce qu'**il comprend au moins un module d'émission d'alerte supplémentaire, réalisant, dans son état activé, l'envoi d'un message de type et de contenu paramétrables déterminés, vers une destination paramétrable déterminée, par des moyens (R2) supplémentaires d'émission et de réception en liaison avec un réseau local ou le réseau Internet.

23. Dispositif d'alerte selon l'une des revendications 13 à 22, **caractérisé en ce que** le module d'analyse comprend une pluralité de modules d'évaluation, correspondant chacun à un scénario d'évaluation et associés chacun à au moins un module de surveillance d'une variable d'alerte et à un module d'évaluation.

## Patentansprüche

1. Warnverfahren, das von einer tragbaren Vorrichtung in einer Bewegungssituation in Association mit wenigstens einem Telekommunikationsnetz ausgeführt wird, wobei die tragbare Vorrichtung wenigstens Verarbeitungsmittel (UC), Speichermittel (MEM) und Mittel (A, DM, M) zum Empfangen oder Senden von Daten über das Netz umfasst, wobei das Verfahren wenigstens die folgenden Schritte umfasst:
- einen Schritt (ETP0081) des Initialisierens von wenigstens einem Analysemodul (101), initialisiert in Abhängigkeit von Daten, die einen bestimmten Nutzungskontext repräsentieren und wenigstens Daten beinhalten, die wenigstens einen Zustand von wenigstens einem Sensor repräsentieren, wobei das Analysemodul wenigstens ein Beurteilungsmodul umfasst, das wenigstens eine Warnvariable (V01, V02, V03) beurteilt,
- einen Schritt (ETP084) des Initialisieren von wenigstens einem Überwachungsmodul (M01, M02, M03), das in Abhängigkeit von dem Nutzungskontext initialisiert wird, wobei das Überwachungsmodul die beurteilte Warnvariable in einem Zustand regelt, der den Zustand dieses Sensors oder eines anderen Sensors (CM01, CM02) in Assoziation mit dem Überwachungsmodul repräsentiert,
- einen Schritt (ETP0083) des Initialisierens von wenigstens einem Modul (102, 1021, 1022) zum Senden einer Warnung, initialisiert in Abhängigkeit von dem Nutzungskontext, gesteuert in Abhängigkeit von der Beurteilung der Warnvariablen durch das Beurteilungsmodul, das bei seiner Aktivierung wenigstens das Senden einer Warnnachricht durch die Mittel zum Senden von Daten über das Netz oder durch andere Sendemittel (T102) auslöst,
- einen Schritt des Regelns, vor der Aktivierung, wenigstens des Beurteilungs-, Überwachungs- oder Warnungssendemoduls, wobei das Regeln durch die Benutzeroberfläche erfolgt,
- einen Schritt (ETP011) des Aktivierens des Analysemoduls und seines Beurteilungsmoduls,
- einen Schritt des Regelns der Warnvariablen durch das mit dem Kontext assoziierte Überwachungsmodul,
- einen Schritt (ET014) des Aktivierens des Warnungssendemoduls, wenn das Beurteilungsmodul einen Notfallzustand erkennt.

2. Warnverfahren nach Anspruch 1, **dadurch gekennzeichnet, dass** der Schritt des Initialisierens des Analysemoduls (101), des Überwachungsmoduls oder des Warnmoduls, realisiert in Abhängigkeit von wenigstens einem den Nutzungskontext repräsentierenden Datenelement, das Vergleichen von wenigstens diesem Datenelement mit Nutzungsdaten in Assoziation mit Modulen einer Bibliothek beinhaltet.

3. Warnverfahren nach Anspruch 2, **dadurch gekennzeichnet, dass** es einen Schritt des Archivierens des Analysemoduls, des Überwachungsmoduls bzw. des Alarmsendemoduls in der Modulbibliothek beinhaltet, der das Speichern von Daten, die das Analysemodul (101), das Überwachungsmodul bzw. das Warnungssendemodul (102) repräsentieren, assoziiert mit Daten, die einen vom Benutzer eingegebenen Namen repräsentieren, oder mit Daten, die den Nutzungskontext repräsentieren, beinhaltet.

4. Warnverfahren nach einem der Ansprüche 1 bis 3, **dadurch gekennzeichnet, dass** das Aktivieren des Analysemoduls parallel zum Speichern, durch ein Lernmodul (106), von Computerereignissequenzen erfolgt, die Zustände des Analyse-, Überwachungs- und Warnungssendemoduls repräsentierten, im Phaseneinklang mit den Zuständen von Warnvariablen.

5. Warnverfahren nach einem der Ansprüche 1 bis 4, **dadurch gekennzeichnet, dass** auf den Schritt (ETP011) des Aktivierens des Analysemoduls und seines Beurteilungsmoduls wenigstens ein Schritt des Regelns des Analysemoduls (101), des Überwachungsmoduls oder des Warnungssendemoduls (102) folgt, in Abhängigkeit von:
- einem über eine Benutzeroberfläche (IHM) eingegebenen manuellen Parametrierungsbefehl, oder
- einem automatischen Parametrierungsbefehl, erzeugt durch das Lernmodul in Abhängigkeit von einer über die Benutzeroberfläche eingegebenen Aufhebung des Notfallzustands.

6. Warnverfahren nach Anspruch 5, **dadurch gekennzeichnet, dass** das Regeln des Analysemoduls das Regeln seines Beurteilungsmoduls beinhaltet durch:
- Wählen eines Beurteilungsprogramms in einer Programmbibliothek, oder
- Parametrieren des gewählten Beurteilungsprogramms, oder
- Assoziieren von einem oder mehreren Warnungssendemodulen, gesteuert in Abhängigkeit von der Beurteilung, oder
- Regeln von einer oder mehreren Referenzen von gelesenen Warnvariablen am Eingang des Beurteilungsprogramms.

7. Warnverfahren nach Anspruch 5 oder 6, dadurch gekenntzeichnet, dass das Regeln des Überwachungsmoduls Folgendes beinhaltet:
- Regeln einer Referenz eines überwachten Sensors, oder
- Wählen einer vorläufigen Verarbeitungs funktion, die die Warnvariable regelt, oder
- Parametrieren der Vorverarbeitungsfunktion, oder
- Regeln einer Verzögerung der Warnungsauslösung.

8. Warnverfahren nach einem der Ansprüche 5 bis 7, **dadurch gekennzeichnet, dass** das Regeln des Warnungssendemoduls Folgendes beinhaltet:
- Wählen des Nachrichtentyps, oder
- Regeln des Inhalts der Nachricht, oder
- Regeln von wenigstens einer Warnvariablenreferenz (V01, V02, V03) oder einer Folge von Computerereignissen, eingefügt in den Inhalt der gesendeten Warnnachricht bei der Aktivierung des Warnungssendemoduls, oder
- Regeln des Zielorts der Nachricht, oder
- Regeln von Freigabe- oder Ausgabeparametern eines Warnzustands.

9. Warnverfahren nach Anspruch 4, **dadurch gekennzeichnet, dass** auf den Schritt des Aktivierens des Warnungssendemoduls (102) wenigstens die folgenden Schritte folgen:
- ein Schritt des Deaktivierens des Beurteilungsmoduls (C102),
- ein Schritt des automatischen Regelns, durch das Lernmodul (106), des Beurteilungsmoduls (101) oder seines assoziierten Überwachungsmoduls oder seines assoziierten Alarmsendemoduls (102),
- ein Schritt des Aktivierens des Beurteilungsmoduls (C102).

10. Warnverfahren nach Anspruch 9, **dadurch gekennzeichnet, dass** dem Schritt des Deaktivierens des Beurteilungsmoduls (C102) wenigstens die folgenden Schritte vorangehen:
- ein Schritt des Sendens einer Vorwarnnachricht zur Benutzeroberfläche (IHM),
- ein Schritt des Wartens auf eine Eingabe einer Aufhebung des Notfallzustands durch die Benutzeroberfläche,
- ein Schritt des Erkennens der Eingabe einer Aufhebung, gefolgt von dem Schritt des Deaktivierens des Beurteilungsmoduls (C102).

11. Warnverfahren nach einem der Ansprüche 1 bis 10, **dadurch gekennzeichnet, dass** das Beurteilungsmodul (C102) wenigstens Folgendes ausführt:
- einen Schritt des Verzögerns, vor der Validierung des Notfallzustands, beginnend sobald die geprüfte Variable einen parametrierbaren Schwellenwert überschreitet, der unterbrochen wird, falls die geprüfte Variable unter den Schwellenwert zurückkehrt.

12. Warnverfahren nach einem der Ansprüche 1 bis 11, **dadurch gekennzeichnet, dass** die Aktivierung des Warnungssendemoduls einen Telefozaanruf mittels eines Mikrofons, eines Lautsprechers oder einer Kamera auslöst.

13. Tragbare Vorrichtung in einer Bewegungssituation in Assoziation mit einem Telekommunikationsnetz, die wenigstens Folgendes umfasst:
- Mittel (A, DM, M) zum Empfangen oder Senden von Daten über das Netz,
- Verarbeitungsmittel (UC),
- Speichermittel (MEM) zum Speichern eines Nutzungskontexts, der Daten enthält, die wenigstens einen Zustand von wenigstens einem Sensor repräsentieren, wobei die Verarbeitungsmittel in Assoziation mit den Speichermitteln so ausgelegt sind, dass sie wenigstens Folgendes realisieren:
ein Analysemodul (101), initialisiert in Abhängigkeit vom Nutzungskontext und gesteuert durch ein Steuermodul (103) in einem aktiven oder ruhenden Zustand,
wobei das Analysemodul wenigstens ein Beurteilungsmodul (C102) umfasst, das wenigstens ein Warnungssendemodul steuert, das in Abhängigkeit von dem Kontext, in Abhängigkeit von wenigstens einer Warnvariablen, geregelt in einem einen Sensor repräsentierenden Zustand, durch ein Überwachungsmodul (M01, M02, M03) geregelt wird, das in Abhängigkeit von dem Kontext initialisiert wird,
wenigstens ein Modul aus Analysemodul, Warnungssendemodul und Überwachungsmodul, das vom Benutzer vor seiner Aktivierung geregelt wird,
wobei das Analysemodul (101) in seinem aktiven Zustand durch sein Beurteilungsmodul (C102) die Aktivierung des Warnungssendemoduls steuert, wenn die geprüfte(n) Variable(n) einen Notfallzustand repräsentiert/-en, wobei das aktivierte Warnungssendemodul (102) das Senden von wenigstens einer Warnnachricht durch die Sendemittel von Daten über das Netz oder über andere Sendemittel auslöst.

14. Tragbare Vorrichtung nach Anspruch 13, **dadurch gekennzeichnet, dass** sie ein Parametrierungsmodul (104) umfasst, das das Beurteilung-, überwachungs- oder Alarmsendemodul regelt, wenn das Analysemodul im Ruhezustand ist, wobei das Parametrierungsmodul pilotgesteuert wird durch:
- eine Benutzeroberfläche (IHM), oder
- ein Lernmodul (106), oder
- ein Fernsteuermodul (105).

15. Tragbare Vorrichtung nach Anspruch 14, **dadurch gekennzeichnet, dass** das Lernmodul (106) in seinem aktiven Zustand Folgen von Computerereignissen speichert, die Zustände des Überwachungs-, Beurteilungs- und Alarmsendemoduls repräsentieren, im Phaseneinklang mit den Zuständen von Warnvariablen (V01, V02, V03).

16. Tragbare Vorrichtung nach Anspruch 15, **dadurch gekennzeichnet, dass** ein Fernsteuersignal von dem Fernsteuermodul (105) empfangen wird, das das Analysemodul (101) im aktiven oder ruhenden Zustand pilotsteuert.

17. Tragbare Vorrichtung nach einem der Ansprüche 13 bis 16, **dadurch gekennzeichnet, dass** sie eine Bibliothek von Analyse-, Beurteilung-, Überwachungs- oder Alarmsendemodulen, die jeweils für eine bestimmte Aktivität dediziert sind, oder von Analyse-, Beurteilungs-, Überwachungs- oder Alarmsendemodulen umfasst, die jeweils mit Daten in Association mit einer Initialisierung gesichert werden, die den Nutzungskontext beim Sichern repräsentieren.

18. Tragbare Vorrichtung nach einem der Ansprüche 13 bis 17, **dadurch gekennzeichnet, dass** sie wenigstens einen Umgebungssensor umfasst, der zu seinem assoziierten Überwachungsmodul ein Signal sendet, das eine physikalische oder chemische Charakteristik einer Umgebung repräsentiert, in der sich die tragbare Vorrichtung befindet.

19. Tragbare Vorrichtung nach einem der Ansprüche 13 bis 18, **dadurch gekennzeichnet, dass** sie wenigstens einen biometrischen Sensor umfasst, der zu seinem assoziierten Überwachungsmodul ein Signal sendet, das eine physiologische Charakteristik des Benutzers der tragbaren Vorrichtung repräsentiert.

20. Tragbare Vorrichtung nach einem der Ansprüche 13 bis 19, **dadurch gekennzeichnet, dass** sie wenigstens einen Lokalisierungssensor (LOC) umfasst, der zu seinem assoziierten Überwachungsmodul ein Signal sendet, das einen geografischen Ort repräsentiert, an dem sich die tragbare Vorrichtung befindet.

21. Tragbare Vorrichtung nach einem der Ansprüche 13 bis 20, **dadurch gekennzeichnet, dass** sie wenigstens einen Sensor für die Anwesenheit einer persönlichen Karte umfasst, die zu ihrem assoziierten Überwachungsmodul ein Signal sendet, das die Anwesenheit der persönlichen Karte in der Nähe der tragbaren Vorrichtung repräsentiert.

22. Tragbare Vorrichtung nach einem der Ansprüche 13 bis 21, **dadurch gekennzeichnet, dass** sie wenigstens ein zusätzliches Warnungssendemodul umfasst, das in seinem aktivierten Zustand eine Nachricht des Typs und des Inhalts bestimmter Parameter zu einem bestimmten parametrisierbaren Ziel sendet, mit zusätzlichen Sende- und Empfangsmittel (R2) in Verbindung mit einem lokalen Netz oder dem Internet.

23. Warnvorrichtung nach einem der Ansprüche 13 bis 22, **dadurch gekennzeichnet, dass** das Analysemodul mehrere Beurteilungsmodule umfasst, jeweils entsprechend einem Beurteilungsszenario und jeweils assoziiert mit wenigstens einem Überwachungsmodul für eine Warnvariable und mit einem Beurteilungsmodul.

## Claims

1. Method for alerting executed by a portable device in a mobility situation associated with at least one telecommunications network, the portable device comprising at least processing means (UC), storing means (MEM) and means (A, DM, M) for receiving or transmitting data over the network, the method comprising at least:
- a step (ETP0081) for initialising at least one analysing module (101), initialised as a function of data representative of a predetermined context of use comprising at least data representative of at least one state of at least one sensor, the analysing module comprising at least one evaluation module evaluating at least one alert variable (V01, V02, V03),
- a step (ETP084) for initialising at least one surveillance module (M01, M02, M03) initialised as a function of the context of use, the surveillance module setting the evaluated alert variable in a state representative of the state of said sensor or of another sensor (CM01, CM02) associated with the surveillance module,
- a step (ETP0083) for initialising at least one module (102, 1021, 1022) for transmission of an alert initialised as a function of the context of use, controlled as a function of the evaluation of the alert variable by the evaluation module and triggering, during its activation, at least the sending of an alert message by the means for transmitting data over the network or by other means (T102) for transmission,
- a step for setting prior to the activation at least the module for evaluation, surveillance or transmission of an alert, the setting being carried out via the user interface,
- a step (ETP011) for activation of the analysing module and its evaluation module,
- a step for setting the alert variable by the surveillance module associated with the context,
- a step (ET014) for activation of the module for transmission of an alert when the evaluation module detects a state of emergency.

2. Method for alerting according to claim 1, **characterised in that** the step for initialising the analysing module (101), the surveillance module or the alert transmission module, carried out as a function of at least one item of data representative of the context of use, comprises the comparison of at least that item of data with initialisation data associated with modules of a library.

3. Method for alerting according to claim 2, **characterised in that** it comprises a step for archiving the analysing module, the surveillance module or respectively the alert transmission module, in the library of modules, comprising the storage of data representative of the analysing module (101), the surveillance module or respectively the alert transmission module (102), associated with data representative of a name entered by the user or of data representative of the context of use.

4. Method for alerting according to one of claims 1 to 3, **characterised in that** the activation of the analysing module is carried out concomitant with the storage, by a learning module (106), of sequences of computer events representative of the states of the module for analysing, surveillance and transmission of an alert, in phase synchronisation with the states of the alert variables.

5. Method for alerting according to one of claims 1 to 4, **characterised in that** the step (ETP011) for activating the analysing module and its evaluation module is followed by at least one step for setting the analysing module (101),the surveillance module or the alert transmission module (102), as a function of:
- a manual configuration command entered via a user interface (IHM), or
- an automatic configuration command produced by the learning module as a function of a cancellation of the state of emergency entered via the user interface.

6. Method for alerting according to claim 5, **characterised in that** the setting of the analysing module comprises the setting of its evaluation module by:
- a choice of an evaluation programme in a library of programmes, or
- a configuration of the chosen evaluation programme, or
- an association of one or more alert transmission modules controlled as a function of the evaluation, or
- a setting of one or more references for alert variables read on entry of the evaluation programme.

7. Method for alerting according to claim 5 or 6, **characterised in that** the setting of the surveillance module comprises:
- a setting of a reference for a surveilled sensor, or
- a choice of a preliminary processing function setting the alert variable, or
- a configuration for the preliminary processing function, or
- a setting of a delay in triggering an alert.

8. Method for alerting according to one of claims 5 to 7, **characterised in that** the setting of the alert transmission module comprises:
- a choice of the type of message, or
- a setting of the contents of the message, or
- a setting of at least one reference for an alert variable (V01, V02, V03) or a sequence of computer events inserted in the contents of the alert message that is sent during the activation of the alert transmission module, or
- a setting of the destination of the message, or
- a setting of parameters for enabling or outputting a state of alert.

9. Method for alerting according to claim 4, **characterised in that** the step for activating the alert transmission module (102) is followed by at least:
- a step for deactivating the evaluation module (C102),
- a step for automatic setting, by the learning module (106), of the evaluation module (101) or of its associated surveillance module or of its associated alert transmission module (102),
- a step for activating the evaluation module (C102).

10. Method for alerting according to claim 9, **characterised in that** the step for deactivating the evaluation module (C102) is preceded by at least:
- a step for sending a warning message, to the user interface (IHM),
- a step for waiting for an entry cancelling the state of emergency, via the user interface,
- a step for detection of the cancellation entry followed by the step for deactivation of the evaluation module (C102).

11. Method for alerting according to one of claims 1 to 10, **characterised in that** the evaluation module (C102) executes at least:
- a step for delaying prior to validation of the state of emergency, starting if the variable being tested exceeds a configurable threshold, and stopping if the variable being tested returns to below the threshold.

12. Method for alerting according to one of claims 1 to 11, **characterised in that** the activation of the alert transmission module triggers a telephone call with the aid of a microphone, a loudspeaker or a camera.

13. Portable device in a mobility situation associated with a telecommunications network, comprising at least:
- means (A, DM, M) for receiving or transmitting data over the network,
- processing means (UC),
- storage means (MEM)
storing a context of use comprising data representative of at least one state of at least one sensor, the processing means associated with the storage means being arranged so as to produce at least:
an analysing module (101), initialised as a function of the context of use and controlled in an active or dormant state by a control module (103),
the analysing module comprising at least one evaluation module (C102) controlling at least one alert transmission module initialised as a function of the context, as a function of at least one alert variable set in a state representative of a sensor, by a surveillance module (M01, M02, M03) initialised as a function of the context,
at least one module from the analysing module, the alert transmission module and the surveillance module being set by the user prior to its activation, the analysing module (101), in its active state, controlling through its evaluation module (C102), the activation of the alert transmission module if the variable or variables tested
are representative of a state of emergency, the activated alert transmission module (102) triggering the sending of at least one alert message by the means for transmitting data over the network or by other means for transmission.

14. Portable device according to claim 13, **characterised in that** it comprises a configuration module (104) setting the module for evaluation, surveillance or alert transmission, when the analysing module is in its dormant state, the configuration module being piloted by:
- a user interface (IHM), or
- a learning module (106),or
- a remote control module (105).

15. Portable device according to claim 14, **characterised in that** the learning module (106) in its active state stores sequences of computer events representative of the states of the modules for surveillance, evaluation and alert transmission, in phase synchronisation with the states of the alert variables (V01, V02, V03).

16. Portable device according to claim 15, **characterised in that** a remote control signal is received by the remote control module (105) which pilots the analysing module (101) in the active or dormant state.

17. Portable device according to one of claims 13 to 16, **characterised in that** it comprises a library of modules for analysing, evaluation, surveillance or alert transmission each dedicated to a predetermined activity or of modules for analysing, evaluation, surveillance or alert transmission each backed up with associated initialisation data representative of the context of use at the time of back-up.

18. Portable device according to one of claims 13 to 17, **characterised in that** it comprises at least one environmental sensor communicating, to its associated surveillance module, a signal representative of a physical or chemical characteristic of an environment in which the portable device is located.

19. Portable device according to one of claims 13 to 18, **characterised in that** it comprises at least one biometric sensor communicating, to its associated surveillance module, a signal representative of a physiological characteristic of the user of the portable device.

20. Portable device according to one of claims 13 to 19, **characterised in that** it comprises at least one locating sensor (LOC) communicating, to its associated surveillance module, a signal representative of a geographical location in which the portable device is located.

21. Portable device according to one of claims 13 to 20, **characterised in that** it comprises at least one sensor for detecting the presence of a personal card communicating, to its associated surveillance module, a signal representative of the presence of the personal card in proximity to the portable device.

22. Portable device according to one of claims 13 to 21, **characterised in that** it comprises at least one module for transmitting a supplementary alert, carrying out, in its activated state, the sending of a message of predetermined configurable type and content to a predetermined configurable destination, by supplementary means (R2) for transmitting and receiving linked to a local network or the Internet network.

23. Warning device according to one of claims 13 to 22, **characterised in that** the analysing module comprises a plurality of evaluation modules, each corresponding to a scenario for evaluation and each associated with at least one module for surveillance of an alert variable and with an evaluation module.
